(19) **Europäisches Patentamt**
**European Patent Office**
**Office européen des brevets**

(11) **EP 4 509 052 B1**

(12) **EUROPEAN PATENT SPECIFICATION**

(45) Date of publication and mention
of the grant of the patent:
**15.07.2026 Bulletin 2026/29**

(21) Application number: **24194085.7**

(22) Date of filing: **12.08.2024**

(51) International Patent Classification (IPC):
*A61B 5/287* (2021.01)     *A61B 5/361* (2021.01)
*A61B 5/367* (2021.01)     *A61B 5/00* (2006.01)
*A61B 18/14* (2006.01)     *A61B 18/00* (2006.01)

(52) Cooperative Patent Classification (CPC):
**A61B 5/287; A61B 5/361; A61B 5/367;**
**A61B 5/7203; A61B 5/7257; A61B 5/726;**
**A61B 18/1492;** A61B 2018/00357;
A61B 2018/00577; A61B 2018/00839

(54) **SYSTEM FOR ELECTROGRAM-BASED LESION ASSESSMENT**

SYSTEM ZUR ELEKTROGRAMMBASIERTEN LÄSIONSBEURTEILUNG

SYSTÈME D'ÉVALUATION DE LÉSION BASÉE SUR UN ÉLECTROGRAMME

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **16.08.2023 US 202363532966 P**

(43) Date of publication of application:
**19.02.2025 Bulletin 2025/08**

(73) Proprietor: **St. Jude Medical, Cardiology Division,**
**Inc.**
**St. Paul, MN 55117 (US)**

(72) Inventors:
• **AFONSO, Valtino X.**
**Oakdale, 55128 (US)**

• **BIRD, Nathaniel**
**Minneapolis, 55414 (US)**
• **FISH, Jeffrey**
**Maple Grove, 55311 (US)**
• **PEDERSON, Brian D.**
**New Braufels, 78132 (US)**

(74) Representative: **Mathys & Squire**
**32 London Bridge Street**
**The Shard**
**London SE1 9SG (GB)**

(56) References cited:
**US-A1- 2015 208 942**     **US-A1- 2019 320 927**

**Description**

**BACKGROUND**

This disclosure is related generally to systems and methods of assessing lesion formation and specifically to lesion assessment based on monitored intracardiac electrogram (EGM) signals.

Cardiac ablation is a medical procedure utilized to generate lesions on the cardiac tissue to disrupt or modify the propagation of electrical signals within the heart. That is, the cardiac lesions prevent the propagation of electrical signals and if correctly located will prevent the propagation of unwanted or irregular electrical signals. Various types of ablation technologies may be utilized to generate the desired lesions. Including radiofrequency (RF) ablation, cryoablation, electroporation, and others. The goal of each type of ablation treatment method is the same however, the generation of lesions that disrupt or block electrical signals.

Typically, during a cardiac ablation procedure, a 2D or 3D map is generated and displayed to physicians, illustrating the location of the ablation catheter within the heart and the locations of the catheter when ablation therapy was delivered. However, no feedback is received regarding the lesions themselves or the effectiveness of the generated lesions. It would be beneficial to provide a system and method of monitoring and receiving feedback regarding lesion formation.

US2019/320927 describes a system and method for mapping cardiac activity. US2015/208942 describes a double bipolar configuration for atrial fibrillation annotation.

**SUMMARY**

The invention is set out in the appended claims.

According to one aspect, a system includes a catheter having at least a first electrode and a second electrode located at a distal end of the catheter and an electronic control unit (ECU) configured to perform a plurality of steps/functions. In some embodiments, the ECU is configured to receive at least a first intracardiac electrogram measured by either the first electrode, the second electrode, or the first and second electrode, wherein the first intracardiac electrogram is received from the first electrode and a second intracardiac electrogram signal is received from the second electrode, wherein the first intracardiac electrogram and the second intracardiac electrogram are unipolar electrograms, detect an activation timepoint and select a roving activation interval (RAI) based on the detected activation timepoint, apply a continuous wavelet transform (CWT) to the first intracardiac electrogram within each RAI to generate a power spectrum response, a phase spectrum response, or both a power spectrum and a phase spectrum response. The ECU is configured to calculate one or more per-RAI metrics based on the power spectrum response, phase spectrum response, or both the power spectrum response and the phase spectrum response and further configured to calculate a per-lesion metric based on the one or more per-RAI metrics. The ECU calculates one or more per-RAI metrics by: creating a bipolar electrogram based on the first and second intracardiac electrograms, wherein the CWT is applied to the bipolar electrogram and at least one of the first and second unipolar electrograms; locating a dominant frequency timepoint based on a power spectrum response generated by applying the CWT to the bipolar electrogram; calculating a maximum power value associated with one of the first unipolar electrogram or second unipolar electrogram based on a power spectrum response generated by applying the CWT to either the first or second unipolar electrogram and the dominant frequency timepoint; and calculating a per-RAI unipolar energy metric for at least one of the first unipolar electrogram or second unipolar electrogram based on the calculated maximum power. The ECU also causes a lesion assessment marker to be displayed on a display based on the calculated per-lesion metric, wherein the lesion assessment marker provides an indication of lesion formation.

DESCRIPTION OF THE DRAWINGS

Fig. 1 is a diagrammatic view of a catheter device in communication with a visualization system according to some embodiments.

Fig. 2 is a block diagram of components utilized by the visualization system to generate lesion assessment metrics based on monitoring electrogram signals according to some embodiments.

Fig. 3 is an isometric view of a distal tip of a catheter utilized to delivery ablation treatment and monitor electrogram signals according to some embodiments.

Fig. 4 is a flowchart illustrating steps utilized to calculate one or more per-lesion metrics based on monitored electrogram signals according to some embodiments.

Fig. 5 is a flowchart illustrating steps utilized to calculate one or more per-RAI metrics based on first and second unipolar electrogram signals monitored according to some embodiments.

Fig. 6 is a flowchart illustrating steps utilized to calculate one or more per-RAI metrics based on a bipolar electrogram signals monitored according to some embodiments.

Fig. 7 is a flowchart illustrating generation of lesion assessment markers based on comparisons of pre-ablation per-lesion metrics with intra-ablation and/or post-ablation per-lesion metrics according to some embodiments.

Fig. 8 is an exemplary graphical user interface for displaying lesion assessment markers to a physician/technician according to some embodiments.

Fig. 9 is an electrocardiogram graph illustrating detection of activation timepoints in a reference electrocardiogram signal according to some embodiments.

Fig. 10 is an electrogram graph of a single roving activation interval (RAI) associated with a surface electrocardiogram, a first unipolar electrogram signal and a second unipolar electrogram signal according to some embodiments.

Fig. 11 is an electrogram graph illustrating calculation of a bipolar electrogram signal based on a first and second unipolar electrogram signal according to some embodiments.

Fig. 12 illustrates an electrogram graph of a bipolar electrogram signal (top) and a power spectrum graph of the bipolar electrogram signal (bottom) according to some embodiments.

Fig. 13 illustrates an electrogram graph of a unipolar electrogram signal (top) and a power spectrum graph of the unipolar electrogram signal (bottom) according to some embodiments.

Fig. 14 illustrates an electrogram graph of a unipolar electrogram signal (top) with Q, R, S points detected and a CWT phase spectrum graph of the unipolar electrogram signal (bottom) with Q, R, S points detected according to some embodiments.

Fig. 15 is an electrogram graph of a unipolar electrogram signal illustrating calculation of Q-R, R-S, and Q-S metrics according to some embodiments.

Fig. 16 is an electrogram graph of a bipolar electrogram signal illustrating calculation of a peak-to-peak metric according to some embodiments.

Figs. 17a-17c are unipolar electrogram graphs (top) and bipolar electrogram graphs (bottom) taken pre-ablation, intra-ablation and post-ablation according to some embodiments.

Figs. 18a-18c are unipole electrogram graphs (top) and power spectrum graphs (bottom) measured pre-ablation, intra-ablation, and post-ablation according to some embodiments.

## DETAILED DESCIRPTION

Disclosed herein is a system and method of assessing lesion formation. The method is not part of the invention. The method includes collecting electrogram signals monitored by one or more electrodes located on the catheter. The electrogram signals may be unipolar electrogram signals or bipolar electrogram signals. Activation timepoints are detected and utilized to select roaming activation intervals (RAI) that include detected depolarizations. For each RAI detected, monitored electrogram signals are analyzed using a continuous wavelet transform (CWT), which analyzes the local frequency response to the monitored electrograms within the RAI. Based on the CWT analysis, one or more metrics are extracted from each RAI interval (referred to as per-RAI metrics). Exemplary per-RAI unipolar metrics include an energy metric and QRS related metrics (e.g., Q-R metric, R-S metric, Q-S metric). Per-RAI bipolar metrics may include a peak-to-peak metric. In some embodiments, the per-RAI metrics may be combined to generate a per-lesion metric. For example, per-RAI metrics of the same type (e.g., energy metric) collected during a given time period (e.g., 3 seconds of therapy application) may be averaged to generate a per-lesion metric. In some embodiments, per-lesion metrics calculated pre-ablation may be compared with per-lesion metrics calculated intra-ablation or post-ablation to make lesion assessment determinations, which may be provided as feedback via a display or interface for review by a physician/technician as lesion assessment markers. In response to lesion assessment feedback, ablation therapy may be modified to generate a desired result. For example, the physician may modify the power delivered to the tissue to modify the ablation response. Other variables associated with the delivery of ablation treatment may also be modified, including determinations of whether a particular site needs additional ablation treatment.

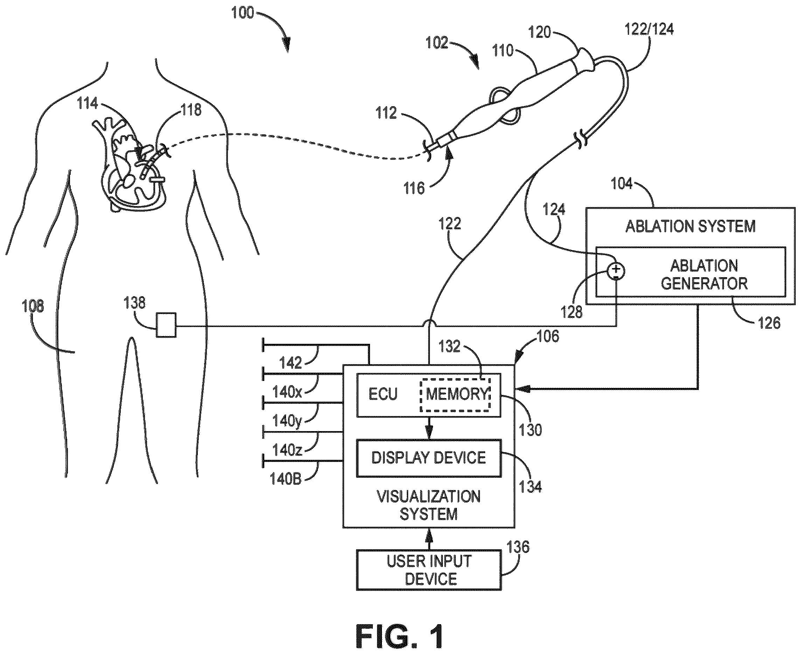

Fig. 1 is a diagrammatic view of a medical system 100 that includes a catheter 102, an ablation system 104, and a visualization system 106. The catheter 102 includes a handle 110, a shaft 112 having a distal end 114 and a proximal end 116, and a cable connector 120 having one or more cables 122, 124 for connecting the catheter to the visualization system 106 and the ablation system 104, respectively. The distal end 114 of catheter 102 includes one or more electrodes 118 for delivering ablation therapy and/or for monitoring intracardiac electrogram signals utilized for assessing lesion formation as described in more detail below. In some embodiments, ablation system 104 includes an ablation generator 126 having an ablation source 128 configured to deliver ablation therapy to the catheter 102 via cable 124. In some embodiments, ablation generator 128 may also be connected to a surface patch electrode 138 to disperse RF ablation current provided to the one or more electrodes located on the catheter. In other embodiments, other types of ablation treatment may be utilized that does not require a surface patch electrode 138. In some embodiments, visualization system 106 includes electronic control unit (ECU) 130, memory 132, display device 134, and user input device 136. Visualization system 106 is connected to the catheter 102 via cable 122 to receive inputs from the one or more electrodes 118 (or other sensors) located at the distal end 114 of the catheter 102. In addition, the visualization system 106 may be connected to one or more patch electrodes 142, $140_x$, $140_y$, $140_z$, and $140_B$. In some embodiments, visualization system 106 utilizes the surface patch electrode 142 to monitor electrograms. In some embodiments, described in more detail below, the electrograms monitored by the surface patch electrode 142 (or any of the other surface patch electrodes) may be utilized as a system reference value.

With continued reference to FIG. 1, catheter 102 is provided for examination, diagnosis, and/or treatment of internal body tissues. In an exemplary embodiment, catheter 102 comprises an ablation catheter. It should be understood, however, that catheter 102 is not limited to a particular ablation catheter. For example, catheter 102 may be an RF ablation catheter, an irrigated ablation catheter, an electroporation ablation catheter, cryoablation catheter, ultrasound ablation catheter, etc. Although not shown in Fig. 1, if necessary, catheter 102 may further include additional components such as a fluid source and/or pump for providing irrigation to the distal end 114 of the catheter 102.

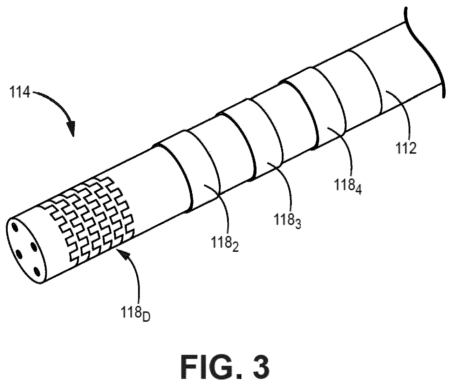

In addition, catheter 102 is electrically connected to ablation system 104 to allow for the delivery of ablation energy. Catheter 102 may include a cable connector or interface 120, handle 110, shaft 112 having a proximal end 116 and distal end 114 (as used herein, "proximal" refers to a direction toward the end of catheter 102 near the clinician, and "distal" refers to a direction away from the clinician and (generally) inside the body of a patient), and one or more electrodes 118 mounted in or on shaft 112 of catheter 102. In an exemplary embodiment, electrodes 118 are disposed at or near distal end 114 of shaft 112, with at least one electrode comprising an ablation electrode disposed at the extreme distal end 114 of shaft 112 (an exemplary embodiment of which is shown in Fig. 3). In some embodiments, additional electrodes such as ring electrodes may be located at various locations along shaft 112. In other embodiments, the distal end 114 of catheter 102 may include various other geometries, including for example a basket geometry comprising a plurality of splines carrying one or more electrodes 118. In yet other embodiments, the distal end 114 of the catheter 102 may include other geometries (e.g., spiral) including one or more electrodes 118.

As described in more detail below, electrodes 118 may be utilized to deliver ablation treatment. For example, in some embodiments the ablation source 128 is configured to provide ablative energy via cable 124 to one or more of the electrodes 118 (for example, the distal most electrode 118). The ablative energy may be radio-frequency (RF) energy or an electroporation voltage utilized to provide electroporation ablation. In other embodiments, other types of ablative therapy may be delivered via the electrodes or via other components.

In some embodiments, localization/navigation includes the generation of impedance fields within the patient's body via the one or more pairs of patch electrodes 140x, 140y, 140z, and $140_B$. Voltages measured by the one or more electrodes 118 located at the distal end 114 of the catheter 102 are communicated to the visualization system 106 via cable 122 and utilized to determine the location of the electrodes within the patient's body in what is referred to as an impedance-based localization system. The location of the electrodes 118 (or more generally, the distal end 114 of the catheter 102) may be displayed visually on display device 134 to aid a physician/technician in guiding the distal end 114 of the catheter to a desired location. In other embodiments, other types of localization/navigation may be utilized such as magnetic-based localization systems or fluoroscopic-based localization systems.

In addition, one or more of the electrodes 118 may be utilized to monitor intracardiac electrogram signals. As described in more detail below, the intracardiac electrogram signals may include unipolar electrogram signals, bipolar electrogram signals, or a combination of both unipolar and bipolar electrogram signals. A unipolar electrogram signal is measured by a single electrode with reference to a ground electrode (e.g., patch electrode 140B). For example, a unipolar electrogram measured by a most distal electrode may be designated as unipole D electrogram, and the electrogram measured by the second most distal electrode may be designated as unipole 2 electrogram. A bipolar electrogram is measured by two electrodes relative to one another. For example, a bipolar measurement between the most distal electrode and the second most distal electrode may be designated the bipole D2 electrogram. In some embodiments, a bipolar electrogram is calculated by subtracting a first unipolar electrogram (e.g., unipole D electrogram) from a second unipolar electrogram (e.g., unipole 2 electrogram) to generate a bipole electrogram (designated in this example as the bipole D-2 electrogram). In some embodiments, the one or more electrogram signals are communicated to the visualization system 106 via cable 122 for analysis. In some embodiments, analysis includes mapping functions, in which the electrogram signals are acquired and analyzed and combined with imaging to display (via display device 134) the electrical excitation sequence of the cardiac tissue, including the detection of anomalies such as arrhythmias.

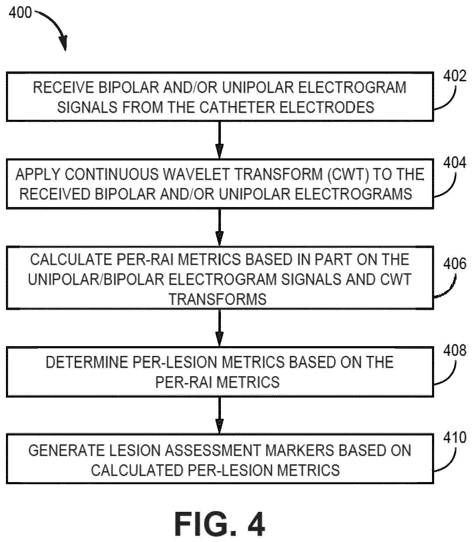

In addition, the one or more electrograms may also be utilized to assess lesion formation prior to, during, and after delivery of ablation treatment (e.g., pre-ablation, intra-ablation, and post-ablation). In this way, electrodes 118 may be utilized for a plurality of different functions, including localization/navigation, ablation delivery, mapping, and/or lesion assessment. As described in more detail with respect Figs. 4-7, in some embodiments lesion assessment is based on analysis of discrete sub-sets of monitored intracardiac electrograms. For example, activation timepoints are detected within a reference signal and utilized to trigger capture of a roving activation interval (RAI) window associated with one or more monitored intracardiac electrogram signals. In addition, visualization system 106 applies a continuous wavelet transform (CWT) to the received RAI electrogram signals, wherein the CWT provides a localized frequency response associated with the monitored signals in the form of a power spectrum and phase spectrum output. Per-RAI metrics are calculated based in part on the power spectrum/phase spectrum outputs. A collection of per-RAI metrics are combined to form a per-lesion metric representative of lesion formation. In some embodiments, pre-ablation per-lesionmetrics are compared with intra-ablation or post-ablation per-lesion metrics to make assessments regarding lesion formation, with one or more lesion assessment markers generated based on the comparison.

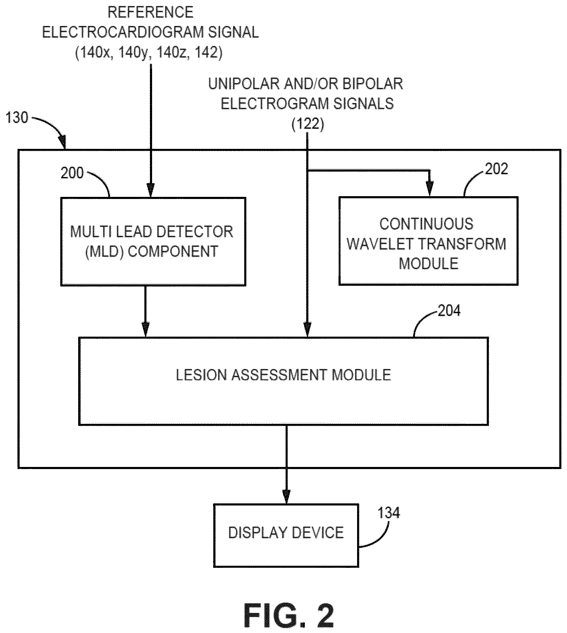

Fig. 2 is a block diagram of components implemented by the visualization system 106 to generate lesion assessment metrics based on monitoring electrograms according to some embodiments. In some embodiments, the components are implemented by the ECU 130 (shown in Fig. 1), and include a multi-lead detector (MLD) component 200, a continuous wavelet transform module 202, and a lesion assessment module 204. In some embodiments, one or more of the MLD detector 200, the CWT module 202 and/or the lesion assessment module 204 are implemented in hardware, software, or a combination of hardware/software components. For example, in some embodiments the ECU 130 executes instructions stored by memory 132 to implement one or more of the MLD detector 200, the CWT module 202 and/or the lesion assessment module 204.

In some embodiments, a reference surface electrogram signal (for example, provided by one or more of the patch electrodes 142 shown in Fig. 1) and at least one of a unipolar or bipolar electrogram signal are received as inputs (for example, unipolar D or unipolar 2 electrograms and/or the bipolar D2 electrogram). In some embodiments, one or more of the electrogram signals are communicated from the one or more electrodes 118 (shown in Fig. 1) to the visualization system 106 via cable 122 connected between the visualization system 106 and the handle 110 of the catheter 102. In some embodiments, the electrograms may be stored in memory 132 for subsequent analysis as described in more detail below.

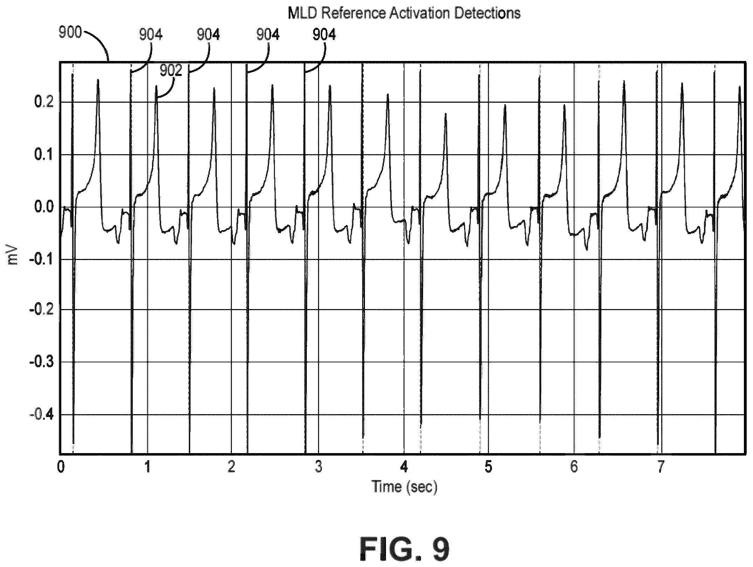

The MLD detector 200 provides activation detection based on a received reference signal. In some embodiments, the reference signal may include one or more of the intracardiac electrograms. In some embodiments, the reference signal may include one or more surface electrocardiograms (ECG) measured by one or more surface ECG patch electrodes 142 rather than an intracardiac electrode. The MLD detector 200 is utilized to detect activations or "beats", characterized by activation timepoints. In some embodiments, the MLD detector 200 provides continuous activation detections. In some embodiments, the MLD detector 200 generates an output consisting of activation timepoints. As described in more detail below, a roving activation interval (RAI) may be established based on the activation time point noting a detected activation. For example, the roving activation interval (RAI) may be established as extending a certain amount of time prior to the detected activation and extending a certain amount of time following the detected activation. As a result, the RAI window selected includes a detected beat. As described below, analysis of the electrogram signal captured within a given RAI window is utilized to assess lesion formation. In some embodiments, one or more parameters associated with the MLD detector 200 may be modified by the user (for example, via user input device 136). In some embodiments, activation detection parameters may be modified, including for example, reference source, reference detection algorithm, reference detection algorithm sensitivity, and RAI high and low time interval curtains. In some embodiments, the MLD detector 200 provides as an output a RAI interval to be analyzed. That is, for each activation detected by the MLD 200, a RAI associated with the activation is selected or output. An example of activations detected by the MLD detector 200 with respect to a reference electrogram signal 902 is provided in Fig. 9. In particular, the dashed vertical lines 904 represent 'activations' detected by the MLD detector 200, each activation associated with a 'beat' detected within the reference signal 902. As described above, the reference signal 902 may be based on reference signals captured by surface patch electrodes or intracardiac electrodes.

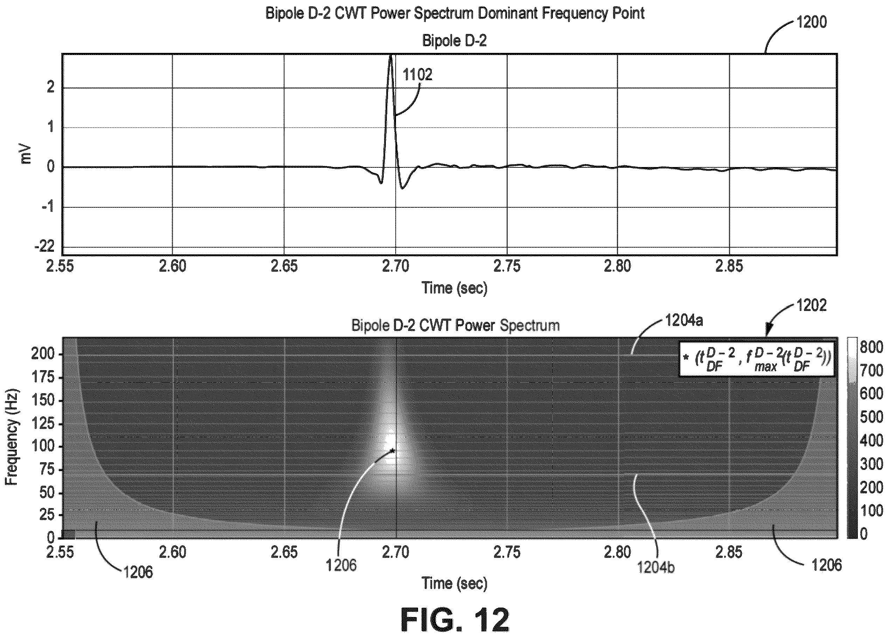

The CWT module 202 receives as input the one or more intracardiac electrogram signals provided within a given RAI window, based on activations detected by the MLD detector 200. The CWT module 202 applies a continuous wavelet transform (CWT) to the electrogram signals within the given RAI window, generating a time-convolution response of the input signal at many frequency scales, resulting in a spectrum of complex values represented as a power spectrum and a phase spectrum. Thatis, the output of the CWT module can be thought of as a localized frequency response. This is in contrast with a Fourier Transform, which is a frequency response that assumes the sample contains an indefinitely repeating example of the signal of interest. The output of the CWT module can be utilized to find the timepoint at which the electrogram signal (defined by the RAI window) is maximally sharp. For example, Fig. 12 illustrates a sample electrogram activation (in this case, a bipolar electrogram 1102) in the electrogram graph 1200 and resulting power spectrum graph 1202 generated by the CWT module 202 (a phase spectrum model may also be generated, not shown). The timepoint atwhich the electrogram signal is maximally sharp is defined by point 1206, defined as the dominant frequency timepoint.

In some embodiments, the lesion assessment module 204 receives the electrograms associated with the RAI windows and the power and phase spectrums generated by the CWT module 202 as inputs and utilizes these inputs to generate one or more metrics, referred to as per-RAI metrics. The type of metrics calculated depend, in part, on the type of electrogram signal being analyzed. For example, unipolar electrogram signals may be analyzed to calculate an energy metric associated with the monitored signal and/or various QRS metrics (e.g., morphologies, defined by QRS metrics, such as Q-R voltage, R-S voltage, and Q-S voltage). Per-RAI metrics extracted from bipolar electrogram signals may include a peak-to-peak metric. In some embodiments, the lesion assessment module 204 combines one or more of the per-RAI metrics calculated to form a per-lesion metric, which is a representative of lesion formation. In some embodiments, the per-lesion metric is an average of a plurality of per-RAI metrics of the same type generated within a given time period (e.g., three seconds). For example, a plurality of per-RAI peak-to-peak metrics may be calculated and averaged to generate a per-lesion metric representative of lesion formation. In other embodiments, the per-lesion metric is a combination of a plurality of different types of per-RAI metrics calculated within a given time period (for example, combination of a per-RAI energy metric, per-RAI QRS metric, and/or per-RAI peak-to-peak metric). In some embodiments, a per-lesion metric associated with a particular location is measured prior to ablative treatment (pre-ablative per-lesion metric), during ablative treatment (intra-ablative per-lesion metric), and post ablative treatment (post-ablative per-lesion metric). In some embodiments, changes in the per-lesion metric (i.e., comparisons of the per-lesions metric from different stages) are utilized to determine or assess lesion formation, as described in more detail below. In some embodiments, the per-lesion metrics and/or comparison of per-lesion metrics are utilized by the lesion assessment module 204 to generate a lesion assessment marker displayed on the display device 134, providing visual feedback to the physician/technician regarding lesion formation. This visual feedback allows the physician to modify the ablative treatment in order to form the desired lesions. For example, the physician may modify the power delivered to the tissue to modify the ablation response or apply additional ablative energy to the selected tissue.

Fig. 3 is an isometric view of a distal end 114 of a catheter 102 utilized to delivery ablation treatment and monitor electrogram signals according to some embodiments. In the embodiment shown in Fig. 3, the distal end 114 of the catheter includes a plurality of electrodes, including distal electrode $118_D$ located at the extreme distal tip of the catheter 102 and a plurality of ring electrodes $118_2$, $118_3$, and $118_4$. In some embodiments, distal electrode $118_D$ is utilized to deliver ablation treatment. In some embodiments, distal electrode $118_D$ is also utilized to monitor intracardiac electrogram signals (either unipolar or bipolar in combination with one of the other electrodes). Likewise, the plurality of ring electrodes $118_2$, $118_3$, $118_4$ may also be utilized to monitor intracardiac electrogram signals. In the exemplary embodiments provided below, a first unipolar electrogram signal is monitored by the distal electrode $118_D$, a second unipolar electrogram signal is monitored by the second electrode $118_2$, and a bipolar electrogram signal is monitored by the electrode pair of distal electrode $118_D$ and second electrode $118_2$. In other embodiments, additional unipolar and bipolar electrogram signals may be monitored by the other electrodes.

Fig. 4 is a flowchart illustrating a method 400 utilized to generate one or more lesion assessment markers based on monitored electrogram signals according to some embodiments. At step 402, bipolar and/or unipolar intracardiac electrogram signals are received from the one or more catheter electrodes. With respect to the exemplary catheter shown in Fig. 3, exemplary unipolar electrogram signals may include a first unipolar electrogram received from electrode $118_D$, a second unipolar electrogram received from electrode $118_2$, and a bipolar electrogram signal received from electrodes $118_D$, $118_2$.

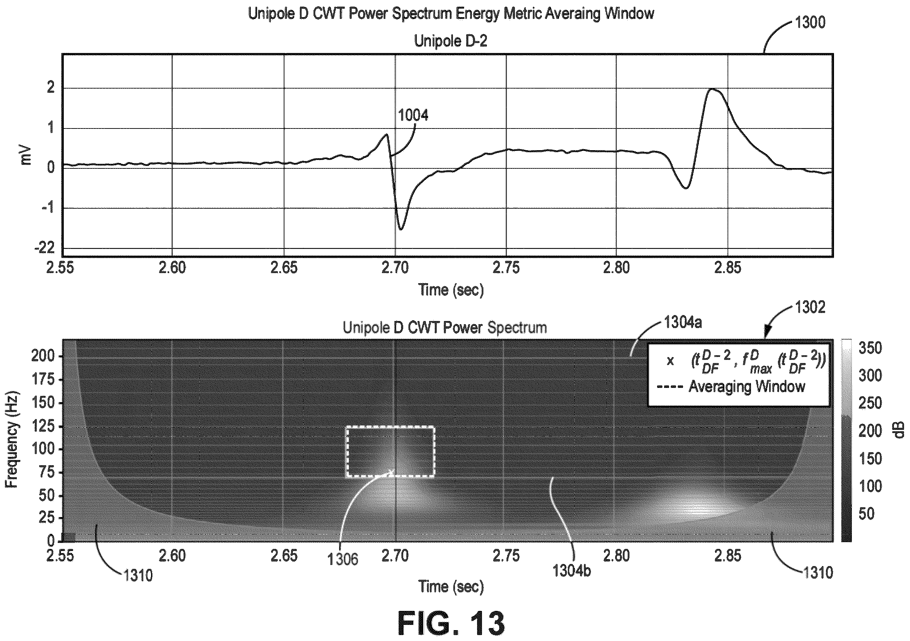

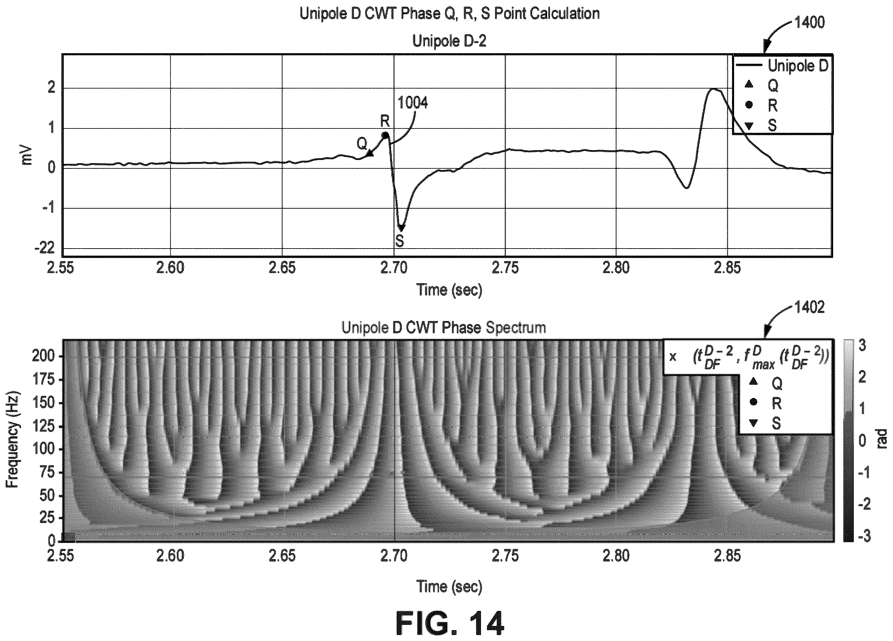

At step 404, a continuous wavelet transform (CWT) is applied to the received electrogram signals. The output of the CWT transform is a complex function that results in both a power spectrum and a phase spectrum component. For example, Fig. 13 illustrates an exemplary power spectrum graph associated with a unipolar electrogram signal and Fig. 14 illustrates an exemplary phase spectrum graph associated with the same unipolar electrogram signal. In some embodiments, the CWT is applied to electrograms associated with a selected RAI window. As discussed above, the RAI window is selected in response to a detected activation timepoint and results in a particular interval (the RAI window) being selected for analysis.

At step 406, unipolar and/or bipolar metrics are calculated based on a combination of the received electrogram signals, the power spectrum output and/or the phase spectrum output. As discussed in more detail below, unipolar metrics may include, for example, an energy metric and/or one or more QRS metrics. Bipolar metrics may include, for example, a peak-to-peak metric. In some embodiments, each metric is calculated with respect to a particular RAI window, and are therefore referred to as per-RAI metrics.

At step 408, a per-lesion metric is determined based on the one or more unipolar/bipolar metrics calculated at step 406. In some embodiments, the plurality of unipolar/bipolar metrics are combined to form the per-lesion metric. In some embodiments, wherein a plurality of per-RAI metrics of the same type (e.g., peak-to-peak) are calculated, the per-lesion metric represents a combining (e.g., averaging) of the plurality of per-RAI metrics. In other embodiments, a plurality of per-RAI metrics of different types are combined to generate the per-lesion metric. In some embodiments, the per-RAI metrics are combined for a given RAI window (e.g., peak-to-peak metric combined with a QRS metric from the same RAI window). In other embodiments, a plurality of per-RAI metrics are combined from a plurality of RAI windows to generate the per-lesion metric. In other embodiments, a plurality of per-RAI metrics from unipolar and bipolar metrics are combined to generate the per-lesion metric. In some embodiments, the per-RAI metrics for a unipolar electrogram are combined with the per-RAI metrics of a bipolar electrogram (e.g., the ratio of the Q-S unipolar metric to the R-S unipolar metric is multiplied with the bipolar peak-to-peak bipolar metric from the same window to arrive at a new per-RAI metric. In still other embodiments, machine learning techniques may be utilized to combine the per-lesion metrics to generate a per-lesion metric.

At step 410, a lesion assessment marker is generated based on one or more per-lesion metrics. For example, in some embodiments, the lesion assessment marker is a visual indicator displayed on a 3D image of the tissue (e.g., heart) that illustrates visually to the physician/technician the lesion status of a particular area. For example, the lesion assessment marker may be a color-coded marker with different colors indicating the progression from "no lesion formed" to "lesion fully formed", with any number of intervening colors utilized to illustrate various partial states of lesion formation.

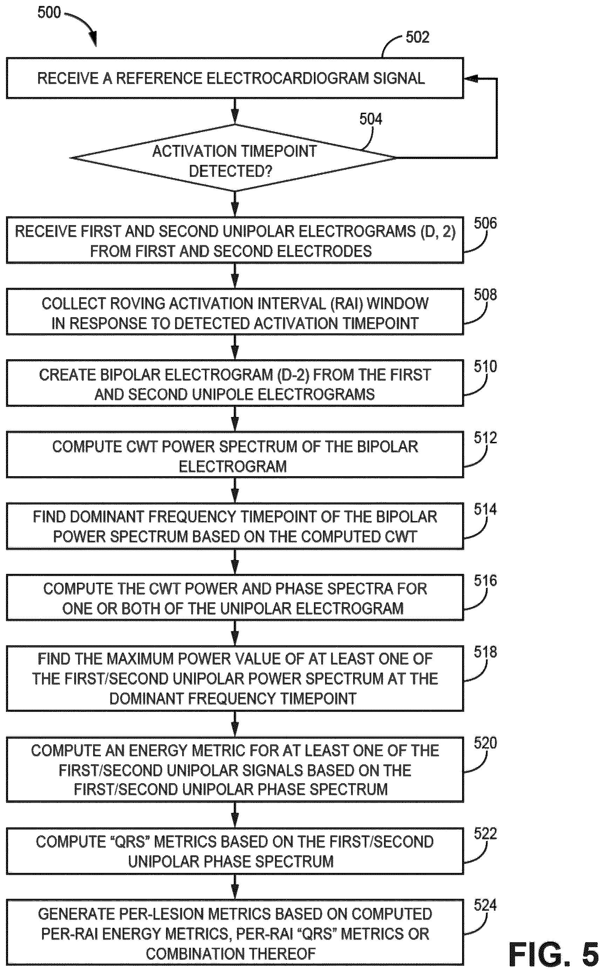

Fig. 5 is a flowchart illustrating a method 500 utilized to calculate one or more unipolar per-RAI metrics and per-lesion metrics based on first and second unipolar electrogram signals monitored according to some embodiments. The exemplary distal tip structure shown in Fig. 3 is utilized to aid in understanding how the signals are utilized, but other catheter configurations may be utilized in other embodiments.

At step 502, a reference electrogram or electrocardiogram is received. In some embodiments, the reference signal is an intracardiac electrogram signal received from one or more of the catheter electrodes. In other embodiments, the reference signal is a surface electrocardiogram received from one or more ECG leads 142 adhered to the patient's skin.

At step 504, activation timepoints are detected within the received reference signal. In some embodiments, an activation timepoint is detected by the MLD detector 200 (shown in Fig. 2). The activation timepoint indicates a detected depolarization of the heart based on the received reference signal (e.g., surface electrocardiogram or intracardiac electrogram). For example, Fig. 9 illustrates a reference electrogram signal 900 and the associated activation timepoints 904 (vertical, dashed lines) identified by the MLD detector 200. In general, an activation time point is detected with respect to each depolarization of the heart. If no activation timepoint is detected, then no further analysis is required and the process continues at step 502 to receive additional reference signals. For example, if no activation timepoint is detected then no depolarization can be analyzed. In some embodiments, MLD detector 200 continually monitors the reference signal to detect activation timepoints. As discussed below, with each detected activation timepoint, a roving activation interval (RAI) is defined and utilized to analyze received electrogram signals.

At step 506 first and second unipolar electrogram signals are received from first and second electrodes located at the distal end of the catheter. For example, with respect to the exemplary embodiment illustrated in Fig. 3, the first unipolar intracardiac electrogram signal may be associated with distal electrode $118_D$, and the second unipolar intracardiac electrogram signal may be associated with the second electrode $118_2$. In some embodiments, additional unipolar electrogram signals may be received from other electrodes (e.g., electrodes $118_3$, $118_4$).

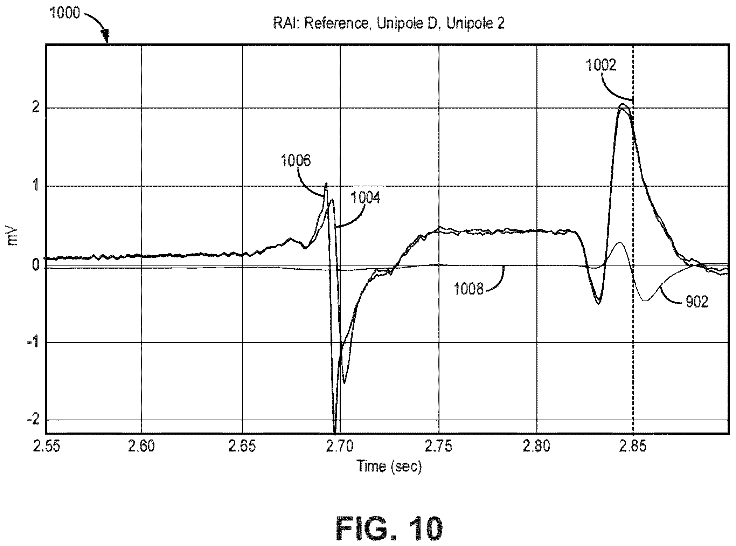

At step 508, a roving activation interval (RAI) window is defined in response to the detected activation timepoint. In some embodiments, the RAI window is selected as an interval of time surrounding the activation timepoint. For example, Fig. 10 illustrates a RAI window 1000 selected based on the activation timepoint 1002 detected with respect to a reference signal 1008. In the embodiment shown in Fig. 10, the RAI window 1000 includes first unipolar intracardiac electrogram 1004 (referred to herein as the unipole D electrogram) and second unipolar intracardiac electrogram 1006 (referred to herein as the unipole 2 electrogram) utilized for subsequent analysis based on the portions of these signals residing within the RAI window 1000. As described above, in some embodiments the RAI window (i.e., the length of the window) may be modified based on one or more parameters, or user-defined, to capture the desired depolarization cycle.

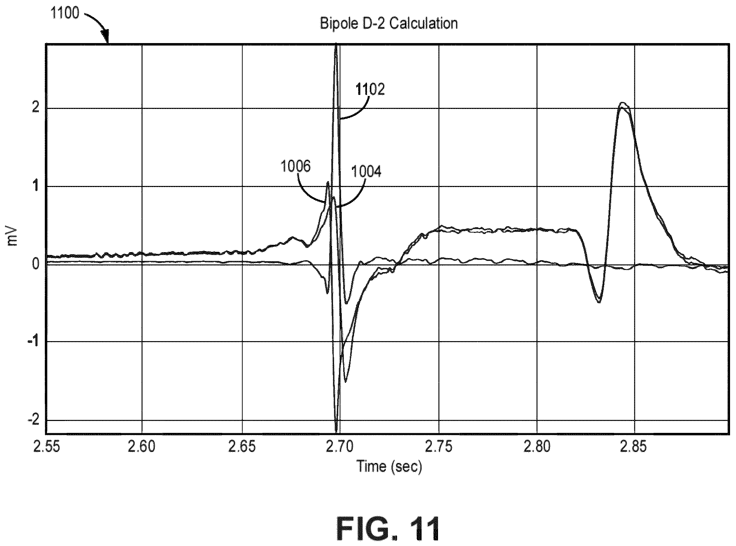

At step 510, a bipolar electrogram is created based on a comparison of the unipole D electrogram 1004 (shown in Fig. 10) and the unipole 2 electrogram 1006. For example, Fig. 11 illustrates a bipolar electrogram 1102 created by subtracting the unipole D electrogram 1004 from the unipole 2 electrogram 1006. For purposes of this example, the bipolar electrogram 1102 created by subtracting the unipole D electrogram 1004 monitored with respect to the distal electrode $118_D$ (unipole D) from the unipole 2 electrogram 1006 monitored with respect to the second electrode $118_2$ (unipole 2) is designated as "bipole D-2". A bipolar electrogram measured between the distal electrode $118_D$ and the second electrode $118_2$ may designated D2 to distinguish from the bipolar electrogram calculated via subtraction of two unipolar electrograms.

At step 512, a continuous wavelet transform (CWT) is applied to the bipolar electrogram D-2 (e.g., electrogram 1102 shown in Fig. 11) to generate a power spectrum representation of the electrogram signal and/or a phase spectra representation of the bipolar electrogram. For example, Fig. 12 illustrates an exemplary embodiment of a power spectrum graph 1202 (bottom graph) generated based on the bipolar electrogram 1102 (shown in electrogram 1200). The x-axis of the CWT power spectrum illustrates time and the *y*-axis illustrates frequency (measured in Hertz (Hz In addition, each pixel is colored to represent the intensity of the frequency response at a given time and at a given frequency (i.e., lighter pixels indicate higher intensity, darker pixels indicate lesser intensity). The grayed-out portion 1206 of the power spectrum graph represents the CWT cone of influence, which indicates values in the CWT power and phase spectrum that are influenced by edge effects. In some embodiments, maximum and minimum frequency responses are defined by the lines 1204a, 1204b to indicate the minimum and maximum frequency scales of interest. As described above, the CWT - unlike the Fourier Transform - represents a local frequency response of the analyzed signal. In the example shown in Fig. 12, the frequency response is aligned in time with the peak/trough of the bipolar electrogram 1102 (approximately 2.70 seconds).

At step 514, a dominant frequency timepoint is determined by the bipolar power spectrum calculated at step 512. In general, the dominant frequency timepoint represents the point in time within the RAI interval corresponding within the highest intensity frequency response. In the exemplary embodiment shown in Fig. 12, the dominant frequency time point associated with the bipolar power spectrum is represented by point 1206, which is defined in space by both a time and a

frequency ( $(t_{DF}^{D-2}, f_{max}^{D-2}(t_{DF}^{D-2})$ , wherein $t_{DF}^{D-2}$ is the time at which the dominant frequency exists and $f_{max}^{D-2}(t_{DF}^{D-2})$ is the dominant frequency (i.e., frequency having the highest intensity response) at the time $t_{DF}^{D-2}$ . In this example, the term "D-2" represents the bipolar electrogram calculated by subtracting the first electrogram measured by the distal electrode $118_D$ from the second unipolar electrogram measured by the second electrode $118_2$, and the term "DF" represents the dominant frequency.

At step 516, a continuous wavelet transform (CWT) is applied to one or more of the unipolar electrogram signals to generate a power spectrum graph and/or phase spectra graph. For example, Fig. 13 illustrates an exemplary embodiment of a CWT power spectrum 1302 (bottom graph) generated based on the unipole D electrogram 1004 (top graph 1300). The x-axis of the CWT power spectrum illustrates time and the y-axis illustrates frequency (measured in Hertz (Hz)). In addition, each pixel is colored to represent the intensity of the frequency response at a given time and at a given frequency (i.e., lighter pixels indicate higher intensity, darker pixels indicate lesser intensity). The grayed-out portion 1310 of the power spectrum graph represents the CWT cone of influence, which indicates values in the CWT power and phase spectrum that are influenced by edge effects. In some embodiments, maximum and minimum frequency responses are defined by the lines 1304a and 1304b to indicate the minimum and maximum frequency scales of interest. As described above, the CWT - unlike the Fourier Transform - represents a local frequency response of the analyzed signal. In the example shown in Fig. 13, the frequency response is aligned in time with the peak/trough of the unipolar electrogram (approximately 2.70 seconds and approximately 2.84 seconds). Fig. 14 provides an example of a phase spectra graph 1402 generated by the CWT applied to the unipole D electrogram 1004. With respect to the phase spectra graph 1402 shown in Fig. 14, the x-axis represents time and the y-axis once again represents frequency, with the shading of the pixels indicating the phase of the signal in units of radians.

At step 518, the maximum power value of at least one of the first/second unipolar power spectrum is determined using the dominant frequency timepoint determined at step 514 with respect to the bipolar power spectrum. For example, in the example described with respect to step 514 and shown in Fig. 12, the dominant frequency timepoint $t_{DF}^{D-2}$ associated with the bipolar electrogram was determined to be at about 2.70 seconds (as shown by point 1206). The maximum power value at this timepoint is determined at this step, as indicated by point 1306 shown in Fig. 13 (which occurs at approximately 2.70 seconds). In the example shown in Fig. 13, the maximum power is associated with a frequency of approximately 75 Hz (again, as indicated by point 1306). Although the unipolar power spectrum 1302 also indicates a frequency response at approximately 2.80 - 2.85 seconds, the subtraction of the first unipolar electrogram signal 1104 from the second unipolar electrogram signal 1106 to generate the bipolar electrogram 1102 indicates that this power spectrum response is associated with a distal event rather than a local event and therefore does not provide useful information for lesion assessment.

At step 520, a per-RAI energy metric is calculated that represents an average energy metric associated with the frequency response surrounding point 1306. In some embodiments, the energy metric is calculated based on the calculated unipolar power spectrum and a frequency range and time interval range selected based on the maximum power value determined at step 518. This is illustrated graphically in Fig. 13 by the bounded box 1308. For example, in some embodiments the frequency range of the averaging window (top and bottom lines associated with the bounded box 1308) is selected by subtracting and adding a predetermined value from the maximum value of the unipolar power spectrum at the dominant frequency timepoint $f_{max}^{D}(t_{DF}^{(D-2)})$ . For example, in some embodiments the averaging window is bounded as follow:

$$[max(70, f_{max}^{D}(t_{DF}^{D-2}) - 50), min(200, f_{max}^{D}(t_{DF}^{D-2}) + 50)] \qquad \text{Eq. 1}$$

wherein the minimum frequency of the averaging window is selected as the maximum of 70 Hz or the dominant frequency timepoint $f_{max}^{D}(t_{DF}^{(D-2)})$ minus some predetermined value (e.g., 50 Hz), and wherein the maximum frequency of the averaging window is selected as the minimum of 200 Hz or the dominant frequency timepoint $f_{max}^{D}(t_{DF}^{(D-2)})$ plus some predetermined value (e.g., 50 Hz). In the embodiment shown in Fig. 13, the frequency range of the averaging window is equal to 70 Hz at a minimum and 125 Hz at a maximum. In some embodiments, the time interval range of the averaging window (left and right lines associated with the bounded box 1308) is selected by subtracting and adding a predetermined value from the dominant frequency timepoint $(t_{DF}^{(D-2)})$ . For example, in some embodiments the averaging window is bounded as follows:

$$[max(t_{min}, t_{DF}^{D-2} - 20), min(t_{max}, t_{DF}^{D-2} + 20)] \qquad \text{Eq. 2}$$

wherein the minimum time range of the averaging window (left line) is selected as maximum of minimum time $t_{min}$ and the dominant frequency timepoint $t_{DF}^{D-2}$ minus some predetermined value (e.g., 20 ms) and the maximum time range of the averaging window (right line) is selected as the minimum of the maximum time $t_{max}$ and the dominant frequency timepoint $t_{DF}^{D-2}$ plus some predetermine value (e.g., 20 ms). In other embodiments, other methods may be utilized to select the time interval range, including basing the time interval range on a width of the dominant frequency peak in the bipolar power spectrum (e.g., width equal to 90% of the maximum power of the power spectrum, etc.).

In some embodiments, having defined the averaging window (e.g., bounding box 1308), the per-RAI energy metric is calculated by summing the energy encompassed by the bounding box 1308. For example, in some embodiments the per-RAI energy metric is calculated as:

$$\text{Energy} = \frac{1}{n} \sum_{f=max\left(70, f_{max}^{D}(t_{DF}^{D-2})-50\right)}^{min\left(200, f_{max}^{D}(t_{DF}^{D-2})+50\right)} \sum_{t=max\left(t_{min}, t_{DF}^{D-2}-20\right)}^{min\left(t_{max}, t_{DF}^{D-2}+20\right)} CWT^{D}(t, f) \text{ dB} \qquad \text{Eq. 3}$$

wherein n is the total number of samples in the summed range. In some embodiments, the energy metric is a floating-point number with units of decibels (dB).

In some embodiments, the per-RAI energy metric calculated (for example using Eq. 3) is provided as a unipolar output representative of lesion formation. In other embodiments, the per-RAI energy metric is modified based on one or more of the per-RAI QRS metrics calculated at step 522. For example, the energy metric may be multiplied by a factor such as *((Q - S)/(R - S))* or *(Q - S)*. In other embodiments, other metrics may be utilized.

At step 522, one or more per-RAI "QRS" metrics are calculated based on the first and/or second unipolar phase spectrum. In some embodiments, the phase spectrum (example shown in Fig. 14, phase spectra graph 1402) is utilized to identify the Q, R, and S timepoints associated with at least one of the unipolar electrogram signals (e.g., unipole D electrogram 1004, unipole 2 electrogram 1006). Having identified the Q, R, and S timepoints, QRS metrics may be calculated based on the measured differences in voltage between the respective Q, R, and S timepoints (e.g, Q-R metric, R-S metric, and Q-S metric).

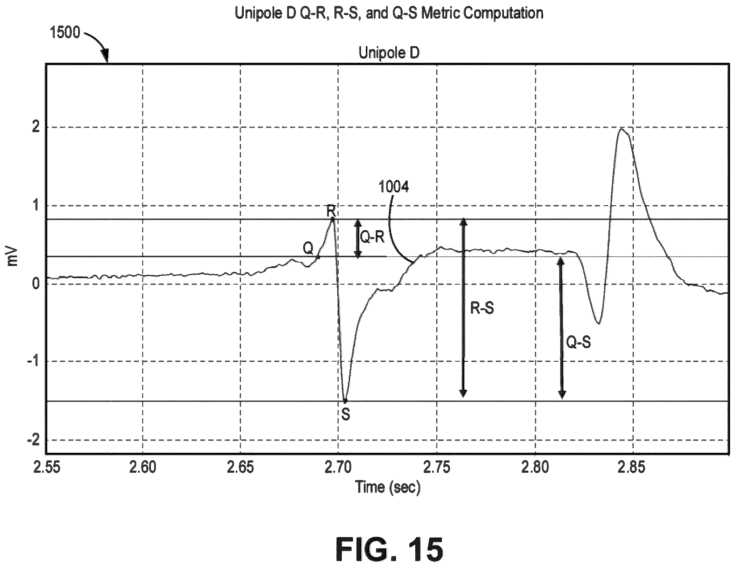

In some embodiments, the Q, R, and S timepoints are identified based on the phase spectrum graph 1402 calculated utilizing the continuous wavelet transform of the one or more unipole electrograms at step 516. For example, in some embodiments, the Q timepoint in the unipole D electrogram 1004 is calculated by starting at the point defined by the bipole dominant frequency timepoint $\left(t_{DF}^{D-2}, f_{max}^{D}(t_{DF}^{D-2})\right)$ and stepping backward in time along the phase spectrum row at frequency scale $f_{max}^{D}(t_{DF}^{D-2})$ until the phase signal crosses the $\pm\pi$ threshold. In some embodiments the R timepoint in the unipole D electrogram 1004 is calculated by starting at a point defined by the Q timepoint $\left(Q, f_{max}^{D}(t_{DF}^{D-2})\right)$ and stepping forward in time along the phase spectrum row at frequency scale $f_{max}^{D}(t_{DF}^{D-2})$ until the phase signal crosses zero. In some embodiments, S timepoint in the unipole D electrogram 1004 is calculated by starting at a point defined by the Q timepoint $\left(Q, f_{max}^{D}(t_{DF}^{D-2})\right)$ and stepping forward in time along the phase spectrum row at frequency scale $f_{max}^{D}(t_{DF}^{D-2})$ until the phase signal crosses $\pm\pi$ threshold. Having identified the Q, R, and S timepoints, the per-RAI QRS metrics are calculated by subtracting the voltage associated with each timepoint as shown visually in Fig. 15, which illustrating the Q-R metric, the R-S metric, and the Q-S metric calculated with respect to the unipole D electrogram 1004.

At step 524, one or more of the per-RAI energy metrics calculated at step 520 and/or the per-RAI QRS metrics calculated at step 522 are utilized to generate a per-lesion metric. In some embodiments, the one or more unipolar per-RAI metrics are calculated with respect to a roving activation interval (RAI), and the unipolar per-lesion metric represents a combination of plurality of per-RAI metrics calculated over a plurality of RAI windows. In some embodiments, the combination of the plurality of per-RAI metrics includes an averaging of the plurality of per-RAI metrics of the same type. In other embodiments, the plurality of per-RAI metrics - of different types - may be combined with one another to generate a per-lesion metric. For example, each per-RAI energy metric may be combined with one or more of the per-RAI QRS metrics to generate a combined per-RAI metric (e.g., via multiplication of the per-RAI energy metric by one of the per-RAI QRS metrics to provide scaling). In some embodiments, a plurality of the scaled metrics may also be combined (e.g., averaged) to generate a per-lesion metric.

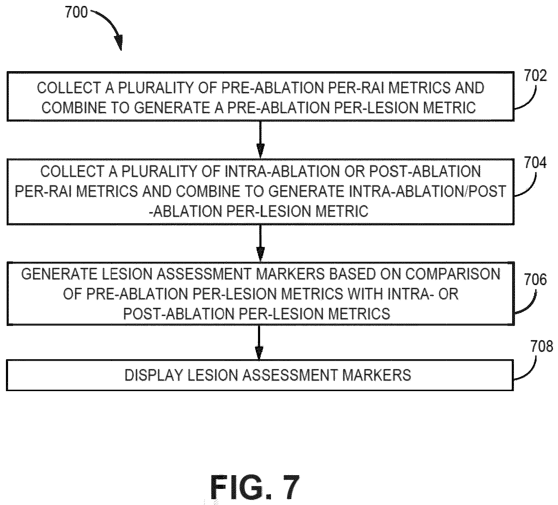

As discussed in more detail with respect to Fig. 7, in some embodiments, a per-lesion metrics measured at different points in time (e.g., pre-ablation, intra-ablation, post-ablation) are compared to one another to assess lesion formation. For example, a pre-ablation per-RAI or per-lesion energy metric may be compared with a post-ablation energy metric, wherein lesion formation causes a decrease in the post-ablation energy metric. Similarly, a pre-ablation per-RAI or per-lesion QRS metric (e.g., Q-R, R-S, or Q-S metric) may be compared with a post-ablation QRS metric, wherein lesion formation causes a decrease in the QRS metrics. In some embodiments, the magnitude of the decrease is representative of lesion formation and can be used to assess lesion formation. In some embodiments, the change in the measured metric (e.g., energy metric, one of the QRS metrics) is compared to a threshold value to assess lesion assessment. In other embodiments, the measured metric is compared to threshold values (without a comparison to previously measured metrics) to assess lesion formation. For example, the threshold value could be based on a physician-defined metric that the physician determines is representative of the beginning of lesion formation.

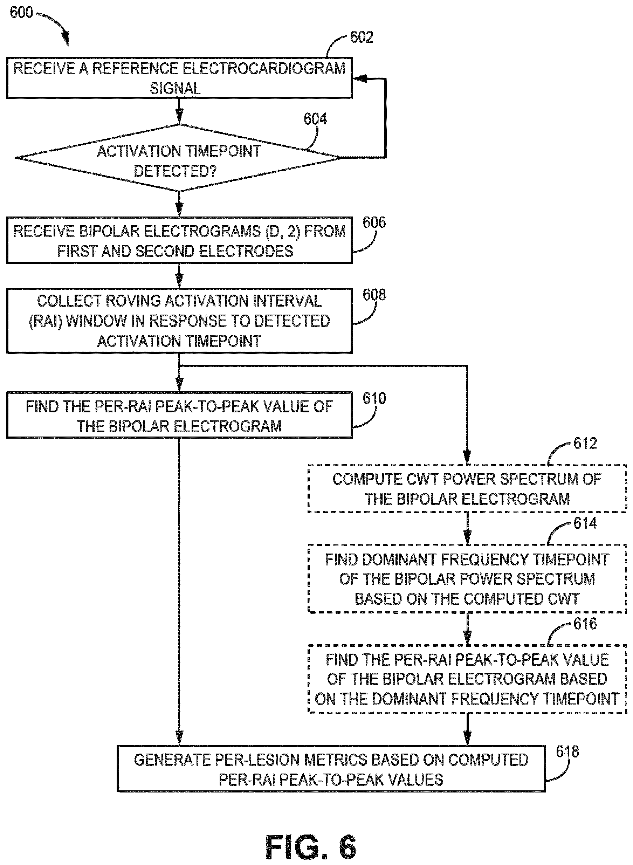

Fig. 6 is a flowchart illustrating a method 600 utilized to calculate one or more bipolar per-RAI metrics and per-lesion metrics based on a bipolar electrogram signal monitored according to some embodiments. The exemplary distal tip structure shown in Fig. 3 is utilized to aid in understanding how the signals are utilized.

At step 602, a reference electrogram is received. In some embodiments, the reference electrogram is an intracardiac signal received from one or more of the catheter electrodes. In other embodiments, the reference electrogram is received from one or more patch electrodes adhered to the patient's skin.

At step 604, activation timepoints are detected within the received reference signal. In some embodiments, an activation timepoint is detected by the MLD detector 200 (shown in Fig. 2). The activation timepoint indicates a detected depolarization of the heart based on the received electrogram signal. For example, Fig. 9 illustrates a reference electrogram signal 902 and the associated activation timepoints 904 (vertical, dashed lines) identified by the MLD detector 200. In general, an activation time point is detected with respect to each depolarization of the heart. If no activation timepoint is detected, then no further analysis is required and the process continues at step 602 to receive additional surface electrocardiograms. For example, if no activation timepoint is detected then no depolarization can be analyzed. In some embodiments, MLD detector 200 continually monitors the reference signal to detect activation timepoints. As discussed below, with each detected activation timepoint, a roving activation interval (RAI) is defined and utilized to analyze received electrogram signals.

At step 606, an intracardiac bipolar electrogram signal is received from an electrode pair located at the distal end of the catheter. For example, with respect to the exemplary embodiment illustrated in Fig. 3, a bipolar electrogram signal is measured by an electrode pair such as distal electrode $118_D$ and second electrode $118_2$. In some embodiments, additional bipolar electrogram signals may be received from other electrode pairs (e.g., electrodes $118_3$, $118_4$).

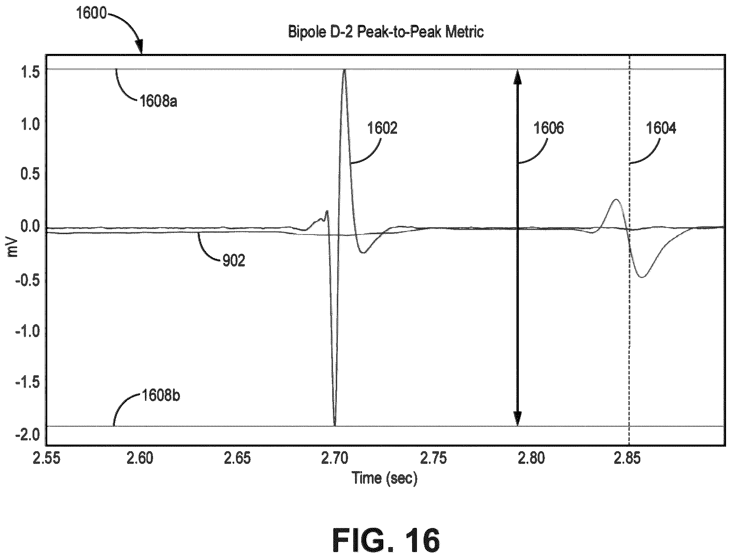

With the activation timepoint detected at step 604, at step 608 a roving activation interval (RAI) window is collected in response to the detected activation timepoint. In some embodiments, the RAI window is selected as an interval of time surrounding the activation timepoint, as described with respect to Fig. 5. For example, Fig. 16 illustrates an electrogram graph 1600 that includes an exemplary RAI of a bipolar electrogram 1602 selected based on activation timepoint 1604 detected with respect to reference signal 902.

At step 610 the bipolar electrogram 1602 is analyzed to detect a peak-to-peak voltage. For example, Figure 16 includes an exemplary bipolar electrogram 1602 with a peak-to-peak bounds 1608a and 1608b. The peak-to-peak voltage 1606 is calculated as the distance between the peak-to-peak bounds 1608a, 1608b. In some embodiments, the peak-to-peak voltage 1606 is calculated based on the maximum voltage detected within the RAI interval and the minimum voltage detected within the RAI window. In some embodiments, the peak-to-peak voltage 1606 includes one or more constraints on the minimum voltage and/or maximum voltage. For example, in some embodiments, a constraint requires the minimum voltage to be detected earlier in time than the maximum voltage. In other embodiments, other constraints may be utilized to ensure the peak-to-peak voltage calculated is representative of the desired characteristics or morphology of the bipolar electrogram 1602.

In some embodiments (illustrated with respect to steps 612, 614, and 616), rather than locate the peak-to-peak voltage across the entire RAI interval, a smaller interval is searched based on the dominant frequency associated with the bipolar electrogram 1602. For example, in some embodiments at step 612 a continuous wavelet transform (CWT) is applied to the bipolar electrogram 1602 to generate a power spectrum graph (and/or a phase spectrum graph). At step 614 the dominant frequency timepoint of the bipolar power spectrum is located based on the power spectrum graph (not shown). As described above, the dominant frequency timepoint corresponds with a particular frequency and timepoint expressed as ( $(t_{DF}^{D2}, f_{max}^{D2}(t_{DF}^{D2})$ , wherein $t_{DF}^{D2}$ is the time at which the dominant frequency exists in the bipolar signal (in this case generated between distal electrode $118_D$ and electrode $118_2$), and $f_{max}^{D2}(t_{DF}^{D2})$ is the dominant frequency (i.e., frequency having the highest intensity response) at the time $t_{DF}^{D2}$. At step 616, a time interval is selected based on the dominant frequency timepoint determined at step 614 and the peak-to-peak voltage is calculated within this time interval. In some embodiments, the time interval selected is shorter than the time interval associated with the RAI window. In some embodiments, selecting a shorter time interval ensures that the minimum and maximum voltage selected as part of the peak-to-peak voltage is based on the desired morphology of the bipolar electrogram 1 (i.e., does not include an extraneous noise signal).

In this way, a per-RAI peak-to-peak metric is calculated, whether via analysis of the bipolar electrogram 1602 or via analysis of the bipolar electrogram 1602 via CWT analysis as described at steps 612, 614, and 616. In some embodiments, the per-RAI peak-to-peak metrics generated according to step 608 and according to steps 610, 612, and 614 may be combined in some way to generate the per-lesion metric. For example, in some embodiments the lesser of the per-RAI peak-to-peak values calculated with respect to each method may be selected as representative. In other embodiments, the per-RAI peak-to-peak metrics may be combined or averaged to generate an average per-RAI peak-to-peak metric.

At step 618, a plurality of per-RAI peak-to-peak metrics calculated at step 610 (or alternatively at step 616, or via a combination of values calculated at step 610 and step 616) are combined or averaged to generate a per-lesion metric. In some embodiments, the lesion assessment metric is generated by averaging a plurality of per-RAI peak-to-peak metrics calculated within a given time interval (e.g., 3 seconds). In other embodiments, other means of combining the per-RAI metrics may be utilized.

Fig. 7 is a flowchart illustrating generation of lesion assessment markers based on comparisons of pre-ablation unipolar and bipolar metrics with intra-ablation and post-ablation unipolar and bipolar metrics according to some embodiments.

At step 702, pre-ablation unipolar/bipolar metrics are collected. As the name implies, pre-ablation unipolar/bipolar metrics are calculated prior to the delivery of ablation treatment to the tissue being monitored. Unipolar metrics may include at least one of an energy metric and a QRS metric (e.g., Q-R metric, R-S metric, Q-S metric) and the bipolar metrics may include, for example, a peak-to-peak voltage metric. In some embodiments, a unipolar/bipolar metrics may be collected with respect to each RAI interval, referred to as a per-RAI metric. In some embodiments, per-RAI metrics are averaged over a selected time period to generate a per-lesion metric. For example, a plurality of per-RAI metrics (e.g., plurality of per-RAI energy metrics) may be collected with respect to a plurality of RAIs within a given time interval (e.g., three seconds) and averaged to generate a per-lesion metric (e.g., per-lesion energy metric).

At step 704, intra-ablation and/or post-ablation unipolar/bipolar metrics are collected. Intra-ablation unipolar/bipolar metrics are collected during the delivery of ablation therapy. Post-ablation unipolar/bipolar metrics are collected following delivery of ablation therapy. In some embodiments, a plurality of per-RAI metrics may be collected for a plurality of RAI intervals. In some embodiments, the plurality of per-RAI metrics are averaged over a selected time period to generate a per-lesion metric. For example, a plurality of per-RAI metrics (e.g., energy metrics) may be collected with respect to a plurality of RAIs within a given time interval (e.g., three seconds) and averaged to generate a per-lesion metric (e.g., per-lesion energy metric).

At step 706, lesion assessment markers are generated based on a comparison of the pre-ablation unipolar/bipolar metrics with the intra-ablation and/or post-ablation unipolar/bipolar metrics. The lesion assessment metric is an indication of lesion formation. In some embodiments, the lesion assessment marker may be expressed as a binary output (lesion formed, no lesion formed). In some embodiments, the lesion assessment metric may be expressed along a sliding scale ranging from no lesion formed to lesion fully formed.

In some embodiments, the change or difference between the pre-ablation metrics and the intra/post-ablation metrics is utilized to generate a lesion assessment metric representative of lesion formation. For example, a pre-ablation per-lesion energy metric may be utilized as a baseline for intra/post-ablation per-lesion energy metrics. In some embodiments, the pre-ablation per-lesion energy metric may be utilized to calculate one or more threshold values utilized to compare the intra/post-ablation per-lesion energy metrics, with the lesion assessment metric being calculated based on the comparison of the intra/post-ablation per-lesion energy metric to the one or more threshold values. For example, the pre-ablation CWT based energy metric may decrease when RF ablation energy is delivered, and this CWT based energy metric may have a lower value in the intra per-lesion metric, and may further decrease in value in the post-ablation metric. In another example, the pre-ablation peak-to-peak bipolar metric may decrease when RF ablation energy is delivered, and this peak-to-peak metric may have a lower value in the intra per-lesion metric, and may further decrease in value in the post-ablation metric.

In some embodiments, a plurality of pre-ablation per-lesion metrics may be collected and compared with a plurality of intra/post-ablation per-lesion metrics. For example, in some embodiments a plurality of pre-ablation per-lesion metrics (e.g., energy metric, QRS metric, peak-to-peak metric) may be compared to corresponding intra/post-ablation per-lesion metrics. In some embodiments, the results of the comparison are combined into an overall lesion assessment metric. For example, if the pre-ablation Q-S metric and the pre-ablation energy metric is large, and the intra-ablation Q-S metric is lower but the intra-ablation energy metric has not changed, an overall intra-ablation lesion assessment metric may be lower than an overall pre-ablation lesion assessment metric based exclusively on the reduction of the Q-S metric from the pre-ablation phase to the intra-ablation phase.

At step 708, the lesion assessment metric generated at step 706 is displayed to the technician/physician. In some embodiments, the lesion assessment metric is displayed visually with respect to a 3D representation of the patient's tissue

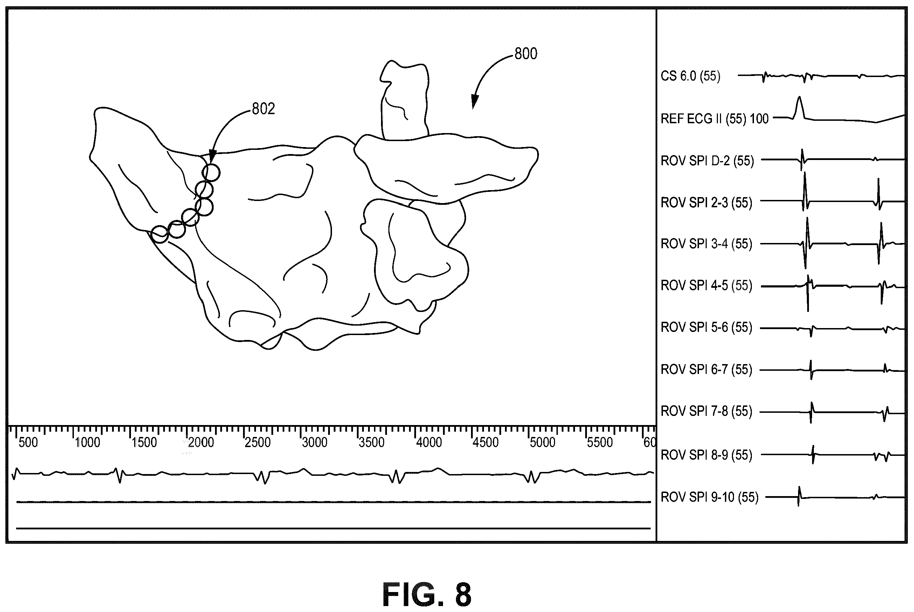

(e.g., heart, pulmonary vein, etc.) to allow the physician/technician to visualize the lesions generated by the ablation process. For example, in the exemplary graphical user interface provided in Fig. 8, a plurality of lesion assessment markers 802 are illustrated with respect to a 3D image of the patient's heart 800. In some embodiments, the color/-shading/size associated with the lesion assessment markers 802 is determined based on the lesion assessment metric calculated at step 706. In this way, the user is provided with feedback regarding each of the lesions formed that may be utilized to determine whether additional treatment is required. In some embodiments, the lesion assessment markers 802 may also be utilized to indicate whether the lesion assessment metric is based on pre-, intra-, or post- ablation lesion assessment metrics.

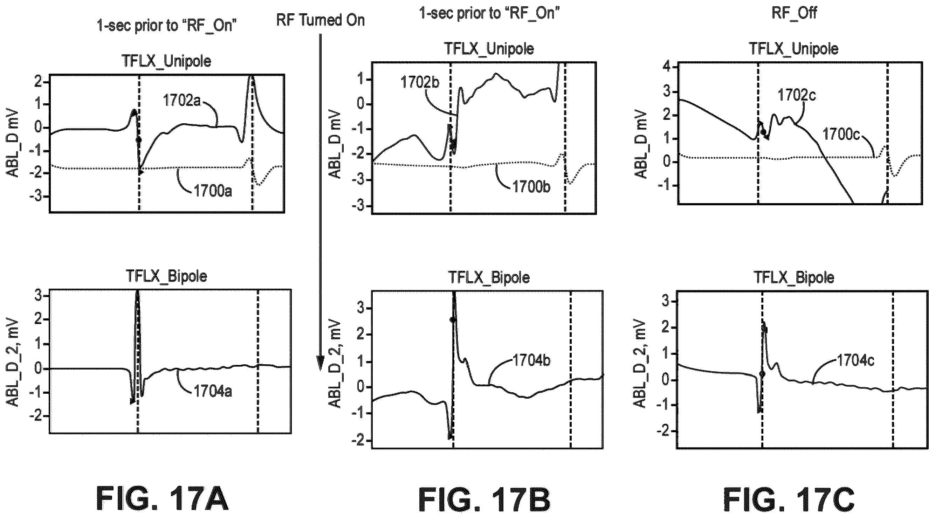

Figs. 17a-17c are unipole electrogram graphs (top) and bipole electrogram graphs (bottom) taken pre-ablation, intra-ablation and post-ablation according to some embodiments. In particular, Fig. 17a illustrates a unipole electrogram 1702a and reference signal 1700a in the top graph and a bipole electrogram 1704a in the bottom graph prior to delivery of ablation treatment (e.g., RF ablation, pulsed field ablation (PFA), etc.). Fig. 17b illustrates a unipole electrogram 1702b and reference signal 1700b in the top graph and a bipole electrogram 1704b in the bottom graph approximately one second after the ablation treatment is turned "ON". Fig. 17c illustrates a unipole electrogram 1702c and reference signal 1700c in the top graph and a bipole electrogram 1704c in the bottom graph following the delivery of ablation treatment.

The top graphs illustrate how the delivery of ablation treatment modifies the unipole electrogram signal. In particular, the shape of unipole electrogram 1702c monitored following delivery of ablation treatment is modified significantly as compared with the unipole electrogram 1702a monitored prior to ablation treatment. Similarly, the shape of the bipole electrogram 1704c monitored following delivery of ablation treatment is modified significantly as compared with the bipole electrogram 1704a monitored prior to ablation treatment. The one or more per-RAI metrics discussed above can be utilized to detect these changes in shape of the unipole and/or bipole electrograms. For example, with respect to the unipole signals 1702a, 1702b, and 1702c, the Q-S metric decreases following delivery of ablation treatment. Likewise, with respect to the bipole signals 1704a, 1704b, and 1704c, the peak-to-peak metric decreases following delivery of ablation treatment.

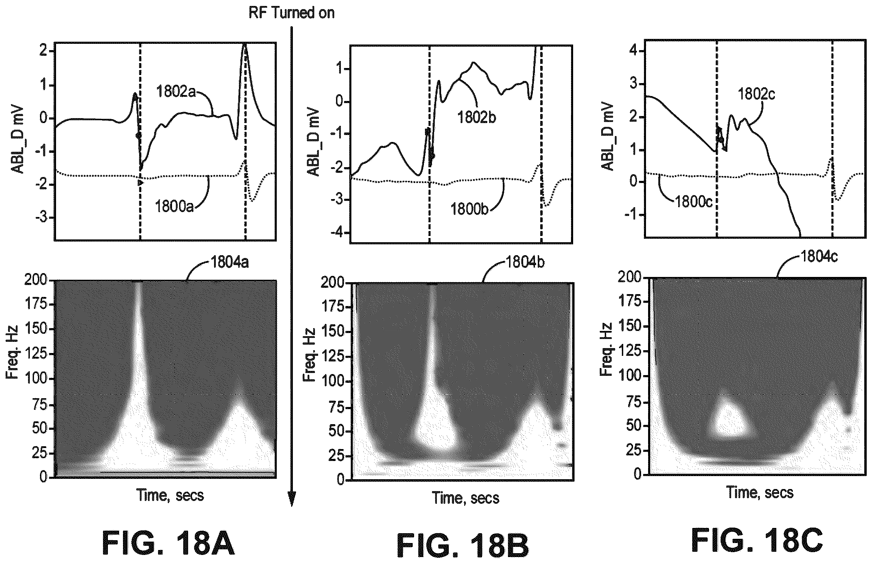

Figs. 18a-18c are unipole electrogram graphs (top) and power spectrum graphs (bottom) taken pre-ablation, intra-ablation, and post-ablation according to some embodiments. In particular, 18a illustrates a unipole electrogram 1802a and reference signal 1800a in the top graph and a power spectrum graph 1804a (bottom) prior to delivery of ablation treatment (e.g., RF ablation, pulsed field ablation (PFA), etc.). Fig. 18b illustrates a unipole electrogram 1802b and reference signal 1800b in the top graph and a power spectrum graph 1804b (bottom) approximately one second after the ablation treatment is turned "ON". Fig. 18c illustrates a unipole electrogram 1802c and reference signal 1800c in the top graph and a power spectrum graph 1804c (bottom) following the delivery of ablation treatment.

The top graphs illustrate how the delivery of ablation treatment modifies the unipole electrogram signal. As shown in Figs. 17a, 17b, and 17c, the unipole electrograms 1802a, 1802b, and 1802c are modified by the delivery of ablation treatment. The corresponding power spectrum graphs 1804a, 1804b, and 1804c reflect these changes to the monitored unipole electrograms 1802a, 1802b, and 1802c. Per-RAI energy metrics generated from the power spectrum graphs 1804a, 1804b, and 1804c can be utilized to assess the changes in the unipole electrograms 1802a, 1802b, and 1802c. In this way, per-RAI energy metrics can be utilized to assess lesion assessment.

Discussion of Possible Embodiments

The following are non-exclusive descriptions of possible embodiments of the present disclosure.

According to one aspect, a method for assessing lesion formation based on monitored electrogram signals includes receiving intracardiac electrogram signals from one or more electrodes located at a distal end of a catheter positioned within a patient, detecting an activation timepoint and selecting a roving activation interval (RAI) based on the detected activation timepoint, and applying a continuous wavelet transform (CWT) to one or more of the received intracardiac electrogram signals within each RAI to generate a power spectrum response, a phase spectrum response, or both a power spectrum and a phase spectrum response. The method may further include calculating one or more per-RAI metrics based on the power spectrum response, phase spectrum response, or both the power spectrum response and the phase spectrum response and calculating a per-lesion metric based on the one or more per-RAI metric. The lesion assessment marker is displayed based on the calculated per-lesion metric, wherein the lesion assessment marker provides an indication of lesion formation.

The method of the preceding paragraph may optionally include, additionally and/or alternatively, any one or more of the following features, steps, configurations and/or additional components.

For example, in some aspects the intracardiac electrogram signal may include a first unipolar electrogram signal and a second unipolar electrogram signal.

In some aspects, the step of calculating one or more per-RAI metrics may include creating a bipole electrogram based on the first and second unipolar electrogram signals, wherein the CWT is applied to the bipole electrogram and at least one of the first and second unipolar electrogram signals, locating a dominant frequency timepoint based on a power spectrum response generated by applying the CWT to the bipole electrogram, calculating a maximum power value associated with one of the first unipolar electrogram signal or second unipolar electrogram signal based on a power spectrum response generated by applying the CWT to either the first or second unipolar electrogram signal and the dominant frequency timepoint, and calculating a per-RAI unipolar energy metric for at least one of the first unipolar electrogram signal or second unipolar electrogram signal based on the calculated maximum power.

In some aspects, the step of calculating one or more per-RAI metrics may include creating a bipole electrogram based on the first and second unipolar electrogram signals, wherein the CWT is applied to the bipole electrogram and at least one of the first and second unipolar electrogram signals, locating a dominant frequency timepoint based on a power spectrum response generated by applying the CWT to the bipole electrogram, locating 'Q', 'R', and 'S' timepoints on at least one of the first and second unipolar electrogram signals based on a phase spectrum response generated by applying the CWT to at least one of the first and second unipolar electrogram signals and the dominant frequency timepoint calculated with respect to the bipole electrogram and calculating one or more per-RAI "QRS" metrics based on the located 'Q', 'R', and 'S' timepoints associated with at least one of the first unipolar electrogram signal or the second unipolar electrogram signal, including at least one of a Q-R voltage, a R-S voltage, and a Q-S voltage.

In some aspects, the intracardiac electrogram signal may include a bipolar electrogram signal.

In some aspects, the step of calculating one or more per-RAI metrics may include determining a per-RAI peak-to-peak voltage associated with the bipolar electrogram signal.

In some aspects, the step of determining a per-RAI peak-to-peak voltage associated with the bipolar electrogram signal may include locating a dominant frequency timepoint based on a power spectrum response generated by applying the CWT to the bipolar electrogram signal and determining a per-RAI peak-to-peak voltage within a subset of the RAI selected based on the dominant frequency timepoint.

In some aspects, calculating a per-lesion metric based on the one or more per-RAI metrics may include collecting a plurality of pre-ablation per-RAI metrics of the same type and averaging the plurality of pre-ablation per-RAI metrics to generate a pre-ablation per-lesion metric, collecting a plurality of intra-ablation per-RAI metrics of the same type and averaging the plurality of intra-ablation per-RAI metrics to generate an intra-ablation per-lesion metric, and comparing the pre-ablation per-lesion metric with the intra-ablation per-lesion metric and generating the lesion assessment marker based on this comparison.

In some aspects, the step of calculating a per-lesion metric based on the one or more per-RAI metrics may include collecting a plurality of pre-ablation per-RAI metrics of the same type and averaging the plurality of pre-ablation per-RAI metrics to generate a pre-ablation per-lesion metric, collecting a plurality of post-ablation per-RAI metrics of the same type and averaging the plurality of post-ablation per-RAI metrics to generate a post-ablation per-lesion metric, and comparing the pre-ablation per-lesion metric with the post-ablation per-lesion metric and generating the lesion assessment marker based on this comparison.

In some aspects, the step of calculating a per-lesion metric based on one or more per-RAI metrics may include collecting a plurality of per-RAI metrics of the same type during therapy application and averaging the plurality of per-RAI metrics to generate a per-lesion metric.

According to another aspect, a system includes a catheter having at least a first electrode and a second electrode located at a distal end of the catheter and an electronic control unit (ECU) configured to perform a plurality of steps/functions. In some embodiments, the ECU is configured to receive at least a first intracardiac electrogram measured by either the first electrode, the second electrode, or the first and second electrode, detect an activation timepoint and select a roving activation interval (RAI) based on the detected activation timepoint, apply a continuous wavelet transform (CWT) to the first intracardiac electrogram within each RAI to generate a power spectrum response, a phase spectrum response, or both a power spectrum and a phase spectrum response. The ECU is configured to calculate one or more per-RAI metrics based on the power spectrum response, phase spectrum response, or both the power spectrum response and the phase spectrum response and further configured to calculate a per-lesion metric based on the one or more per-RAI metrics. The ECU may also cause a lesion assessment marker to be displayed on a display based on the calculated per-lesion metric, wherein the lesion assessment marker provides an indication of lesion formation.

The system of the preceding paragraph may optionally include, additionally and/or alternatively, any one or more of the following features, configurations and/or additional components.

For example, the first intracardiac electrogram may be received from the first electrode and a second intracardiac electrogram signal may be received from the second electrode, wherein the first intracardiac electrogram signal and the second intracardiac electrogram signal are unipolar electrograms.

In some aspects, the ECU calculates one or more per-RAI unipolar metrics by creating a bipolar electrogram based on the first and second intracardiac electrograms, wherein the CWT is applied to the bipolar electrogram and at least one of the first and second unipolar electrograms, locating a dominant frequency timepoint based on a power spectrum response generated by applying the CWT to the bipolar electrogram, calculating a maximum power value associated with one of the first unipolar electrogram or second unipolar electrogram based on a power spectrum response generated by applying the CWT to either the first or second unipolar electrogram and the dominant frequency timepoint, and calculating a per-RAI unipolar energy metric for at least one of the first unipolar electrogram or second unipolar electrogram based on the calculated maximum power.

In some aspects, the ECU may calculate one or more per-RAI unipolar metrics by: creating a bipolar electrogram based on a first intracardiac unipolar electrogram and a second intracardiac unipolar electrogram, wherein the CWT is applied to the bipolar electrogram and at least one of the first and second unipolar electrograms, locating a dominant frequency timepoint based on a power spectrum response generated by applying the CWT to the bipolar electrogram, locating 'Q', 'R', and 'S' timepoints on at least one of the first and second unipolar electrograms based on a phase spectrum response generated by applying the CWT to at least one of the first and second unipolar electrograms and the dominant frequency timepoint calculated with respect to the bipolar electrogram, and calculating one or more per-RAI "QRS" metrics based on the located 'Q', 'R', and 'S' timepoints associated with at least one of the first unipolar electrogram or the second unipolar electrogram, including at least one of a Q-R voltage, a R-S voltage, and a Q-S voltage.

In some aspects, the first intracardiac electrogram may be a bipolar electrogram measured by the first electrode and the second electrode.

In some aspects, the ECU may calculate one or more per-RAI unipolar metrics by determining a per-RAI peak-to-peak voltage associated with the bipolar electrogram.

In some aspects, the ECU may calculate the per-RAI peak-to-peak voltage by: locating a dominant frequency timepoint based on a power spectrum response generated by applying the CWT to the bipolar electrogram, and determining a per-RAI peak-to-peak voltage within a subset of the RAI selected based on the dominant frequency timepoint.

In some aspects, the ECU may calculate a per-lesion metric based on the one or more per-RAI metrics by: collecting a plurality of pre-ablation per-RAI metrics of the same type and averaging the plurality of pre-ablation per-RAI metrics to generate a pre-ablation per-lesion metric, collecting a plurality of intra-ablation per-RAI metrics of the same type and averaging the plurality of intra-ablation per-RAI metrics to generate an intra-ablation per-lesion metric, and comparing the pre-ablation per-lesion metric with the intra-ablation per-lesion metric and generating the lesion assessment marker based on this comparison.

In some aspects, the ECU may calculate a per-lesion metric based on the one or more per-RAI metrics by:collecting a plurality of pre-ablation per-RAI metrics of the same type and averaging the plurality of pre-ablation per-RAI metrics to generate a pre-ablation per-lesion metric, collecting a plurality of post-ablation per-RAI metrics of the same type and averaging the plurality of post-ablation per-RAI metrics to generate a post-ablation per-lesion metric, and comparing the pre-ablation per-lesion metric with the post-ablation per-lesion metric and generating the lesion assessment marker based on this comparison.

In some aspects, the ECU may calculate a per-lesion metric based on the one or more per-RAI metrics by collecting a plurality of per-RAI metrics of the same type during therapy application and averaging the plurality of per-RAI metrics to generate a per-lesion metric.

**Claims**

1. A system comprising:

   a catheter (102) having at least a first electrode and a second electrode (118) located at a distal end (114) of the catheter (102); and
   an electronic control unit, ECU, (130) configured to:

   receive at least a first intracardiac electrogram measured by either the first electrode, the second electrode, or the first and second electrode, wherein the first intracardiac electrogram is received from the first electrode and a second intracardiac electrogram signal is received from the second electrode, wherein the first intracardiac electrogram and the second intracardiac electrogram are unipolar electrograms;
   detect an activation timepoint and select a roving activation interval (RAI) based on the detected activation timepoint;
   apply a continuous wavelet transform, CWT, to the first intracardiac electrogram within each RAI to generate a power spectrum response, a phase spectrum response, or both a power spectrum and a phase spectrum response;

   calculate one or more per-RAI metrics based on the power spectrum response, phase spectrum response, or both the power spectrum response and the phase spectrum response, wherein the ECU calculates one or more per-RAI metrics by:

creating a bipolar electrogram based on the first and second intracardiac electrograms, wherein the CWT is applied to the bipolar electrogram and at least one of the first and second unipolar electrograms;

locating a dominant frequency timepoint based on a power spectrum response generated by applying the CWT to the bipolar electrogram;

calculating a maximum power value associated with one of the first unipolar electrogram or second unipolar electrogram based on a power spectrum response generated by applying the CWT to either the first or second unipolar electrogram and the dominant frequency timepoint; and

calculating a per-RAI unipolar energy metric for at least one of the first unipolar electrogram or second unipolar electrogram based on the calculated maximum power.;

calculate a per-lesion metric based on the one or more per-RAI metrics; and

cause a lesion assessment marker to be displayed on a display (134) based on the calculated per-lesion metric, wherein the lesion assessment marker provides an indication of lesion formation.

2. The system of claim 1, wherein the ECU (130) further calculates one or more per-RAI metrics by:

locating 'Q', 'R', and 'S' timepoints on at least one of the first and second unipolar electrograms based on a phase spectrum response generated by applying the CWT to at least one of the first and second unipolar electrograms and the dominant frequency timepoint calculated with respect to the bipolar electrogram; and

calculating one or more per-RAI "QRS" metrics based on the located 'Q', 'R', and 'S' timepoints associated with at least one of the first unipolar electrogram or the second unipolar electrogram, including at least one of a Q-R voltage, a R-S voltage, and a Q-S voltage.

3. The system of claim 1 or 2, wherein the first intracardiac electrogram is a bipolar electrogram measured by the first electrode and the second electrode, wherein the ECU (130) calculates one or more per-RAI unipolar metrics by determining a per-RAI peak-to-peak voltage associated with the bipolar electrogram.

4. The system of claim 3 wherein the ECU (130) calculates the per-RAI peak-to-peak voltage by:

locating a dominant frequency timepoint based on a power spectrum response generated by applying the CWT to the bipolar electrogram; and

determining a per-RAI peak-to-peak voltage within a subset of the RAI selected based on the dominant frequency timepoint.

5. The system of any of claims 1 to 4, wherein the ECU (130) calculates a per-lesion metric based on the one or more per-RAI metrics by:

collecting a plurality of pre-ablation per-RAI metrics of the same type and averaging the plurality of pre-ablation per-RAI metrics to generate a pre-ablation per-lesion metric;

collecting a plurality of intra-ablation per-RAI metrics of the same type and averaging the plurality of intra-ablation per-RAI metrics to generate an intra-ablation per-lesion metric; and

comparing the pre-ablation per-lesion metric with the intra-ablation per-lesion metric and generating the lesion assessment marker based on this comparison.

6. The system of any of claims 1 to 4, wherein the ECU (130) calculates a per-lesion metric based on the one or more per-RAI metrics by:

collecting a plurality of pre-ablation per-RAI metrics of the same type and averaging the plurality of pre-ablation per-RAI metrics to generate a pre-ablation per-lesion metric;

collecting a plurality of post-ablation per-RAI metrics of the same type and averaging the plurality of post-ablation per-RAI metrics to generate a post-ablation per-lesion metric; and

comparing the pre-ablation per-lesion metric with the post-ablation per-lesion metric and generating the lesion assessment marker based on this comparison.

7. The system of any of claims 1 to 4, wherein the ECU (130) calculates a per-lesion metric based on the one or more per-RAI metrics by:

collecting a plurality of per-RAI metrics of the same type during therapy application and averaging the plurality of per-RAI metrics to generate a per-lesion metric.

**Patentansprüche**

1. System, Folgendes umfassend:

   einen Katheter (102) mit wenigstens einer ersten Elektrode und einer zweiten Elektrode (118), die sich an einem distalen Ende (114) des Katheters (102) befinden; und
   eine elektronische Steuereinheit, ECU, (130), die für Folgendes konfiguriert ist:

   Empfangen wenigstens eines ersten intrakardialen Elektrogramms, das entweder von der ersten Elektrode, der zweiten Elektrode oder der ersten und der zweiten Elektrode gemessen wird, wobei das erste intrakardiale Elektrogramm von der ersten Elektrode empfangen wird und ein zweites intrakardiales Elektrogrammsignal von der zweiten Elektrode empfangen wird, wobei das erste intrakardiale Elektrogramm und das zweite intrakardiale Elektrogramm unipolare Elektrogramme sind;
   Erkennen eines Aktivierungszeitpunkts und auf Grundlage des erkannten Aktivierungszeitpunkts Auswählen eines Roving-Activation-Intervalls (RAI);
   Anwenden einer kontinuierlichen Wavelet-Transformation, CWT, auf das erste intrakardiale Elektrogramm innerhalb jedes RAI, um eine Leistungsspektrumsantwort, eine Phasenspektrumsantwort oder sowohl eine Leistungsspektrums- als auch eine Phasenspektrumsantwort zu erzeugen;

   Berechnen einer oder mehrerer Metriken pro RAI auf Grundlage der Leistungsspektrumsantwort, der Phasenspektrumsantwort oder sowohl der Leistungsspektrumsantwort als auch der Phasenspektrumsantwort, wobei die ECU eine oder mehrere Metriken pro RAI durch Folgendes berechnet:

   Erstellen eines bipolaren Elektrogramms auf Grundlage des ersten und des zweiten intrakardialen Elektrogramms, wobei die CWT auf das bipolare Elektrogramm und wenigstens eines des ersten und des zweiten unipolaren Elektrogramms angewendet wird;
   Lokalisieren eines dominanten Frequenzzeitpunkts auf Grundlage einer Leistungsspektrumsantwort, die durch das Anwenden der CWT auf das bipolare Elektrogramm erzeugt wird;
   Berechnen eines maximalen Leistungswertes, der einem des ersten unipolaren Elektrogramms oder des zweiten unipolaren Elektrogramms zugeordnet ist, auf Grundlage einer Leistungsspektrumsantwort, die durch Anwenden der CWT auf entweder das erste oder das zweite unipolare Elektrogramm und den dominanten Frequenzzeitpunkt erzeugt wird; und
   Berechnen einer unipolaren Energiemetrik pro RAI für wenigstens eines des ersten unipolaren Elektrogramms oder des zweiten unipolaren Elektrogramms auf Grundlage der berechneten maximalen Leistung;

   Berechnen einer Metrik pro Läsion auf Grundlage einer oder mehrerer Metriken pro RAI; und
   Veranlassen, dass auf einer Anzeige (134) auf Grundlage der berechneten Metrik pro Läsion ein Läsionsbewertungsmarkierer angezeigt wird, wobei der Läsionsbewertungsmarkierer einen Hinweis auf die Läsionsbildung bereitstellt.

2. System nach Anspruch **1,** wobei die ECU (130) ferner eine oder mehrere Metriken pro RAI durch Folgendes berechnet:

   Lokalisieren der Zeitpunkte ‚Q', ‚R', und ‚S' auf wenigstens einem des ersten und des zweiten unipolaren Elektrogramms auf Grundlage einer Phasenspektrumsantwort, die durch Anwenden der CWT auf wenigstens eines des ersten und des zweiten unipolaren Elektrogramms erzeugt wird, und des dominanten Frequenzzeitpunkts, der in Bezug auf das bipolare Elektrogramm berechnet wird; und
   Berechnen einer oder mehrerer "QRS-" Metriken pro RAI auf Grundlage der lokalisierten Zeitpunkte ‚Q', ‚R', und ‚S', die wenigstens einem des ersten unipolaren Elektrogramms oder des zweiten unipolaren Elektrogramms zugeordnet sind, einschließlich wenigstens einer einer Q-R-Spannung, einer R-S-Spannung und einer Q-S-Spannung.

3. System nach Anspruch 1 oder 2, wobei das erste intrakardiale Elektrogramm ein bipolares Elektrogramm ist, das von der ersten Elektrode und der zweiten Elektrode gemessen wird, wobei die ECU (130) eine oder mehrere unipolare pro-RAI-Metriken pro RAI berechnet, indem sie eine Peak-Peak-Spannung pro RAI bestimmt, die dem bipolaren Elektrogramm zugeordnet ist.

4. System nach Anspruch 3, wobei die ECU (130) die Peak-Peak-Spannung pro RAI durch Folgendes berechnet:

Lokalisieren eines dominanten Frequenzzeitpunkts auf Grundlage einer Leistungsspektrumsantwort, die durch das Anwenden der CWT auf das bipolare Elektrogramm erzeugt wird; und

Bestimmen einer Peak-Peak-Spannung pro RAI innerhalb einer Teilmenge des RAI, die auf Grundlage des dominanten Frequenzzeitpunkts ausgewählt wird.

**5.** System nach einem der Ansprüche 1 bis 4, wobei die ECU (130) eine Metrik pro Läsion auf Grundlage der einen oder der mehreren Metriken pro RAI durch Folgendes berechnet:

Sammeln einer Mehrzahl von Metriken pro RAI vor der Ablation desselben Typs und Mitteln der Mehrzahl von Metriken pro RAI vor der Ablation, um eine Metrik pro Läsion vor der Ablation zu erzeugen;

Sammeln einer Mehrzahl von Metriken pro RAI innerhalb der Ablation desselben Typs und Mitteln der Mehrzahl von Metriken pro RAI innerhalb der Ablation, um eine Metrik pro Läsion innerhalb der Ablation zu erzeugen; und

Vergleichen der Metrik pro Läsion vor der Ablation mit der Metrik pro Läsion innerhalb der Ablation und Erzeugen des Läsionsbewertungsmarkierers auf Grundlage dieses Vergleichs.

**6.** System nach einem der Ansprüche 1 bis 4, wobei die ECU (130) eine Metrik pro Läsion auf Grundlage der einen oder der mehreren Metriken pro RAI durch Folgendes berechnet:

Sammeln einer Mehrzahl von Metriken pro RAI vor der Ablation desselben Typs und Mitteln der Mehrzahl von Metriken pro RAI vor der Ablation, um eine Metrik pro Läsion vor der Ablation zu erzeugen;

Sammeln einer Mehrzahl von Metriken pro RAI nach der Ablation desselben Typs und Mitteln der Mehrzahl von Metriken pro RAI nach der Ablation, um eine Metrik pro Läsion nach der Ablation zu erzeugen; und

Vergleichen der Metrik pro Läsion vor der Ablation mit der Metrik pro Läsion nach der Ablation und Erzeugen des Läsionsbewertungsmarkierers auf Grundlage dieses Vergleichs.

**7.** System nach einem der Ansprüche 1 bis 4, wobei die ECU (130) eine Metrik pro Läsion auf Grundlage der einen oder der mehreren Metriken pro RAI durch Folgendes berechnet:

Sammeln einer Mehrzahl von Metriken pro RAI desselben Typs während der Therapieanwendung und Mitteln der Mehrzahl von Metriken pro RAI, um eine Metrik pro Läsion zu erzeugen.

## Revendications

**1.** Système comprenant :

un cathéter (102) ayant au moins une première électrode et une deuxième électrode (118) situées au niveau d'une extrémité distale (114) du cathéter (102) ; et

une unité de commande électronique, ECU, (130) configurée pour :

recevoir au moins un électrogramme intracardiaque mesuré par soit la première électrode, la deuxième électrode, ou les première et deuxième électrodes, dans lequel le premier électrogramme intracardiaque est reçu de la première électrode et un deuxième signal d'électrogramme intracardiaque est reçu de la deuxième électrode, dans lequel le premier électrogramme intracardiaque et le deuxième électrogramme intracardiaque sont des électrogrammes unipolaires ;

détecter un point temporel d'activation et sélectionner un intervalle d'activation mobile (RAI) sur la base du point temporel d'activation détecté ;

appliquer une transformée en ondelettes continue, CWT, au premier électrogramme intracardiaque dans les limites de chaque RAI pour générer une réponse spectrale de puissance, une réponse spectrale de phase, ou à la fois une réponse spectrale de puissance et une réponse spectrale de phase ;

calculer une ou plusieurs mesures par RAI sur la base de la réponse spectrale de puissance, de la réponse spectrale de phase, ou à la fois de la réponse spectrale de puissance et de la réponse spectrale de phase, dans lequel l'ECU calcule une ou plusieurs mesures par RAI par :

la création d'un électrogramme bipolaire sur la base des premier et deuxième électrogrammes intracardiaques, dans lequel la CWT est appliquée à l'électrogramme bipolaire et au moins l'un des premier et deuxième électrogrammes unipolaires ;

la localisation d'un point temporel de fréquence dominante sur la base d'une réponse spectrale de puissance générée par l'application de la CWT à l'électrogramme bipolaire ;

le calcul d'une valeur de puissance maximale associée à l'un du premier électrogramme unipolaire ou du deuxième électrogramme unipolaire sur la base d'une réponse spectrale de puissance générée par l'application de la CWT à l'un ou l'autre du premier ou deuxième électrogramme unipolaire et au point temporel de fréquence dominante ; et

le calcul d'une mesure d'énergie unipolaire par RAI pour au moins l'un du premier électrogramme unipolaire ou du deuxième électrogramme unipolaire sur la base de la puissance maximale calculée ;

le calcul d'une mesure par lésion sur la base des une ou plusieurs mesures par RAI ; et

le fait d'amener un marqueur d'évaluation de lésion à être affiché sur un dispositif d'affichage (134) sur la base de la mesure par lésion calculée, dans lequel le marqueur d'évaluation de lésion fournit une indication de formation de lésion.

2. Système selon la revendication 1, dans lequel l'ECU (130) calcule en outre une ou plusieurs mesures par RAI par :

la localisation de points temporels « Q », « R », et « S » sur au moins l'un des premier et deuxième électrogrammes unipolaires sur la base d'une réponse spectrale de phase générée par l'application de la CWT à au moins l'un des premier et deuxième électrogrammes unipolaires et au point temporel de fréquence dominante par rapport à l'électrogramme bipolaire ; et

le calcul d'une ou de plusieurs mesures « QRS » par RAI sur la base de points temporels « Q », « R », et « S » associés à au moins l'un du premier électrogramme unipolaire ou du deuxième électrogramme unipolaire, incluant au moins l'une d'une tension Q-R, d'une tension R-S, et d'une tension Q-S.

3. Système selon la revendication 1 ou 2, dans lequel le premier électrogramme intracardiaque est un électrogramme bipolaire mesuré par la première électrode et la deuxième électrode, dans lequel l'ECU (130) calcule une ou plusieurs mesures unipolaires par RAI par la détermination d'une tension crête à crête par RAI associée à l'électrogramme bipolaire.

4. Système selon la revendication 3, dans lequel l'ECU (130) calcule la tension crête à crête par RAI par :

la localisation d'un point temporel de fréquence dominante sur la base d'une réponse spectrale de puissance générée par l'application de la CWT à l'électrogramme bipolaire ; et

la détermination d'une tension crête à crête par RAI au sein d'un sous-ensemble du RAI sélectionné sur la base du point temporel de fréquence dominante.

5. Système selon l'une quelconque des revendications 1 à 4, dans lequel l'ECU (130) calcule une mesure par lésion sur la base des une ou plusieurs mesures par RAI par :

la collecte d'une pluralité de mesures par RAI pré-ablation du même type et la réalisation de la moyenne de la pluralité de mesures par RAI pré-ablation pour générer une mesure par lésion pré-ablation ;

la collecte d'une pluralité de mesures par RAI intra-ablation du même type et la réalisation de la moyenne de la pluralité de mesures par RAI intra-ablation pour générer une mesure par lésion intra-ablation ; et

la comparaison de la mesure par lésion pré-ablation avec la mesure par lésion intra-ablation et la génération du marqueur d'évaluation de lésion sur la base de cette comparaison.

6. Système selon l'une quelconque des revendications 1 à 4, dans lequel l'ECU (130) calcule une mesure par lésion sur la base des une ou plusieurs mesures par RAI par :

la collecte d'une pluralité de mesures par RAI pré-ablation du même type et la réalisation de la moyenne de la pluralité de mesures par RAI pré-ablation pour générer une mesure par lésion pré-ablation ;

la collecte d'une pluralité de mesures par RAI post-ablation du même type et la réalisation de la moyenne de la pluralité de mesures par RAI post-ablation pour générer une mesure par lésion post-ablation ; et

la comparaison de la mesure par lésion pré-ablation avec la mesure par lésion post-ablation et la génération du marqueur d'évaluation de lésion sur la base de cette comparaison.

7. Système selon l'une quelconque des revendications 1 à 4, dans lequel l'ECU (130) calcule une mesure par lésion sur la base des une ou plusieurs mesures par RAI par :
la collecte d'une pluralité de mesures par RAI du même type pendant l'application de thérapie et la réalisation de la moyenne de la pluralité de mesures par RAI pour générer une mesure par lésion.

FIG. 1

**FIG. 2**

FIG. 3

400

RECEIVE BIPOLAR AND/OR UNIPOLAR ELECTROGRAM
SIGNALS FROM THE CATHETER ELECTRODES — 402

APPLY CONTINUOUS WAVELET TRANSFORM (CWT) TO THE
RECEIVED BIPOLAR AND/OR UNIPOLAR ELECTROGRAMS — 404

CALCULATE PER-RAI METRICS BASED IN PART ON THE
UNIPOLAR/BIPOLAR ELECTROGRAM SIGNALS AND CWT
TRANSFORMS — 406

DETERMINE PER-LESION METRICS BASED ON THE
PER-RAI METRICS — 408

GENERATE LESION ASSESSMENT MARKERS BASED ON
CALCULATED PER-LESION METRICS — 410

# FIG. 4

500

502

RECEIVE A REFERENCE ELECTROCARDIOGRAM SIGNAL

504

ACTIVATION TIMEPOINT DETECTED?

RECEIVE FIRST AND SECOND UNIPOLAR ELECTROGRAMS (D, 2) FROM FIRST AND SECOND ELECTRODES — 506

COLLECT ROVING ACTIVATION INTERVAL (RAI) WINDOW IN RESPONSE TO DETECTED ACTIVATION TIMEPOINT — 508

CREATE BIPOLAR ELECTROGRAM (D-2) FROM THE FIRST AND SECOND UNIPOLE ELECTROGRAMS — 510

COMPUTE CWT POWER SPECTRUM OF THE BIPOLAR ELECTROGRAM — 512

FIND DOMINANT FREQUENCY TIMEPOINT OF THE BIPOLAR POWER SPECTRUM BASED ON THE COMPUTED CWT — 514

COMPUTE THE CWT POWER AND PHASE SPECTRA FOR ONE OR BOTH OF THE UNIPOLAR ELECTROGRAM — 516

FIND THE MAXIMUM POWER VALUE OF AT LEAST ONE OF THE FIRST/SECOND UNIPOLAR POWER SPECTRUM AT THE DOMINANT FREQUENCY TIMEPOINT — 518

COMPUTE AN ENERGY METRIC FOR AT LEAST ONE OF THE FIRST/SECOND UNIPOLAR SIGNALS BASED ON THE FIRST/SECOND UNIPOLAR PHASE SPECTRUM — 520

COMPUTE "QRS" METRICS BASED ON THE FIRST/SECOND UNIPOLAR PHASE SPECTRUM — 522

GENERATE PER-LESION METRICS BASED ON COMPUTED PER-RAI ENERGY METRICS, PER-RAI "QRS" METRICS OR COMBINATION THEREOF — 524

FIG. 5

600

602
RECEIVE A REFERENCE ELECTROCARDIOGRAM
SIGNAL

604
ACTIVATION TIMEPOINT
DETECTED?

606
RECEIVE BIPOLAR ELECTROGRAMS (D, 2) FROM
FIRST AND SECOND ELECTRODES

608
COLLECT ROVING ACTIVATION INTERVAL
(RAI) WINDOW IN RESPONSE TO DETECTED
ACTIVATION TIMEPOINT

610
FIND THE PER-RAI PEAK-TO-PEAK VALUE OF
THE BIPOLAR ELECTROGRAM

612
COMPUTE CWT POWER SPECTRUM OF
THE BIPOLAR ELECTROGRAM

614
FIND DOMINANT FREQUENCY TIMEPOINT
OF THE BIPOLAR POWER SPECTRUM
BASED ON THE COMPUTED CWT

616
FIND THE PER-RAI PEAK-TO-PEAK VALUE
OF THE BIPOLAR ELECTROGRAM BASED
ON THE DOMINANT FREQUENCY TIMEPOINT

618
GENERATE PER-LESION METRICS BASED ON COMPUTED
PER-RAI PEAK-TO-PEAK VALUES

FIG. 6

700

COLLECT A PLURALITY OF PRE-ABLATION PER-RAI METRICS AND COMBINE TO GENERATE A PRE-ABLATION PER-LESION METRIC — 702

COLLECT A PLURALITY OF INTRA-ABLATION OR POST-ABLATION PER-RAI METRICS AND COMBINE TO GENERATE INTRA-ABLATION/POST-ABLATION PER-LESION METRIC — 704

GENERATE LESION ASSESSMENT MARKERS BASED ON COMPARISON OF PRE-ABLATION PER-LESION METRICS WITH INTRA- OR POST-ABLATION PER-LESION METRICS — 706

DISPLAY LESION ASSESSMENT MARKERS — 708

# FIG. 7

FIG. 8

EP 4 509 052 B1

FIG. 9

EP 4 509 052 B1

FIG. 10

FIG. 11

Bipole D-2 CWT Power Spectrum Dominant Frequency Point

**FIG. 12**

EP 4 509 052 B1

FIG. 13

FIG. 14

EP 4 509 052 B1

Unipole D Q-R, R-S, and Q-S Metric Computation

FIG. 15

FIG. 16

**FIG. 17A**

**FIG. 17B**

**FIG. 17C**

EP 4 509 052 B1

RF Turned on

FIG. 18A  FIG. 18B  FIG. 18C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019320927 A

- US 2015208942 A